(19)

Europäisches
Patentamt
European
Patent Office
Office européen
des brevets

(11) **EP 4 265 276 A1**

(12) **EUROPEAN PATENT APPLICATION**
published in accordance with Art. 153(4) EPC

(43) Date of publication:
**25.10.2023 Bulletin 2023/43**

(21) Application number: **21907048.9**

(22) Date of filing: **14.12.2021**

(51) International Patent Classification (IPC):
**A61K 48/00** (2003.01)      **A61K 47/54** (2017.01)
**A61K 47/64** (2017.01)      **A61K 31/7088** (2006.01)
**A61P 35/00** (2006.01)      **C12N 15/113** (2010.01)

(52) Cooperative Patent Classification (CPC):
**A61K 31/7088; A61K 47/54; A61K 47/64;
A61K 48/00; A61P 35/00; C12N 15/113**

(86) International application number:
**PCT/KR2021/018964**

(87) International publication number:
**WO 2022/131744 (23.06.2022 Gazette 2022/25)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **15.12.2020  KR 20200175270**

(71) Applicant: **Seasun Therapeutics
Daejeon 34015 (KR)**

(72) Inventors:
  • **KANG, Yu-Sun
    Gimhae-si, Gyeongsangnam-do 50898 (KR)**

  • **KIM, Hye Joo
    Daejeon 34080 (KR)**
  • **YU, Ji-Yeon
    Cheongju-si, Chungcheongbuk-do 28193 (KR)**
  • **PARK, Hee Kyung
    Daejeon 34034 (KR)**

(74) Representative: **Mewburn Ellis LLP
Aurora Building
Counterslip
Bristol BS1 6BX (GB)**

Remarks:
The complete document including Reference
Table(s) and the Sequence Listing(s) can be
downloaded from the EPO website

(54) **COMPOSITION FOR PREVENTING OR TREATING PANCREATIC CANCER COMPRISING PEPTIDE NUCLEIC ACID COMPLEX**

(57)    The present invention relates to a pharmaceutical composition that is for treating pancreatic cancer and contains a nucleic acid complex as an active ingredient, and more specifically, to a pharmaceutical composition that is for preventing or treating pancreatic cancer and contains a nucleic acid complex in which a bioactive peptide nucleic acid, having a sequence binding to the KRAS gene, and a carrier peptide nucleic acid are complementarily bound. The nucleic acid complex according to the present invention has excellent cell permeability and intracellular activity, and can effectively inhibit the expression of the KRAS gene and subtype genes thereof, and is thus useful for the prevention or treatment of pancreatic cancer.

EP 4 265 276 A1

**Fig.3**

(A)

(B)

(C)

NC : Negative Control        PC : Positive Control
SC : Scramble Control       (Gemcitabine, 80 mg/kg)
                                        PNA 1 : PNA 1_2mg/kg

**Description**

**Technical Field**

The present invention relates to a pharmaceutical composition for preventing or treating pancreatic cancer comprising a nucleic acid complex as an active ingredient and more particularly, to a pharmaceutical composition for preventing or treating pancreatic cancer comprising a nucleic acid complex in which a carrier peptide nucleic acid complementarily binds to a bioactive peptide nucleic acid having a sequence capable of binding to a KRAS gene.

**Background Art**

The pancreas is located in the middle of the body behind the stomach and is about 20 cm long. The pancreas is surrounded by organs such as the stomach, duodenum, small intestine, large intestine, liver, gallbladder, and spleen. The pancreas has a total length of about 15 to 20 cm and a weight of about 100 g, and is divided into the head, body, and tail. The pancreas has an exocrine function of secreting digestive enzymes that decompose carbohydrates, fats, and proteins in ingested food, and an endocrine function of secreting hormones such as insulin and glucagon that regulate blood sugar.

Pancreatic cancer is a tumor mass consisting of cancer cells formed in the pancreas. There are several types of pancreatic cancer. However, pancreatic cancer generally refers to "pancreatic ductal adenocarcinoma" because pancreatic ductal adenocarcinoma, which is derived from pancreatic ductal cells, accounts for about 90% of all pancreatic cancers. Other types of pancreatic cancer include cystic cancer (cystadenocarcinoma), endocrine tumors and the like.

Pancreatic tumors include various types ranging from benign pancreatic tumors that can be treated by surgical resection to malignant pancreatic tumors that have a very poor prognosis. Thereamong, the most common cystic tumors (cysts) may be classified into serous and mucinous cystic tumors, intraductal papillary mucinous tumors, solid papillary tumors, and tumors such as lymphoid epithelial cysts and cystic teratomas. Malignant tumors may be classified into pancreatic exocrine tumors, such as pancreatic ductal adenocarcinoma, acinar cell carcinoma, and neuroendocrine tumor.

Pancreatic cancer has no specific early symptoms, and is difficult to detect early because it has symptoms such as appetite or weight loss, but these symptoms are not unique to pancreatic cancer. In addition, the pancreas is as thin as 2 cm in thickness, is covered only with a membrane, and is readily invaded by cancer and metastases nerve bundles and lymph nodes in an early stage because it is in close contact with the superior mesenteric artery that supplies oxygen to the small intestine and the hepatic portal vein that carries nutrients absorbed from the intestine to the liver. In particular, pancreatic cancer cells grow rapidly.

Pancreatic cancer is the 14th most common cancer in the world, the incidence thereof is remarkably increasing and pancreatic cancer is the 4th leading cause of death from cancer in the United States. Regular diagnosis of pancreatic cancer is necessary because pancreatic cancer does not have specific early symptoms and exhibits clinical symptoms such as weakness, loss of appetite, and weight loss after systemic metastasis has already occurred.

Only 1 to 4% of pancreatic cancer patients exhibit a 5-year survival rate after surgery and has prognosis poor enough to reach a median survival time of 5 months. Treatment for pancreatic cancer is mainly dependent on anticancer therapy because 80 to 90% of pancreatic cancer patients are found in a state in which curative resection is not possible at the time of diagnosis.

Surgical tumor resection is known as the best treatment for pancreatic cancer to date. However, even after surgical resection, metastasis may still occur, and the average survival period without evidence of recurrence is known to be about 212 months after resection. In particular, pancreatic cancer is often diagnosed after the cancer has progressed significantly and chemotherapy or radiation therapy is often ineffective therefor.

Representative anticancer drugs currently used to treat pancreatic cancer include Gemcitabine and Tarceva. Gemcitabine, which is most often used for the treatment of pancreatic cancer, was approved by the FDA in 1997 and is an anticancer drug that can be used for the treatment for early stages of pancreatic cancer. Gemcitabine, which is a nucleoside analogue, replaces cytidine in the DNA replication process to inhibit normal replication and thereby exhibit therapeutic effects for cancer.

Tarceva, which is an oral anticancer drug that inhibits epidermal growth factor receptor (EGFR), inhibits the activity of tyrosine kinase of the HER1/EGFR signaling pathway within cells to block the growth of tumor cells and thereby exhibit therapeutic effects for cancer. Tarceva is known to increase survival rate when used in combination with Gemcitabine.

However, the therapeutic effects of several anticancer drugs, including Gemcitabine and Tarceva, which are known to be effective for pancreatic cancer so far, are extremely low and have a response rate to chemotherapy of only around 15%. In order to improve the prognosis of patients, there is urgent demand for the development of more effective early diagnosis and treatment methods.

KRAS is a protein that plays an important role in the differentiation, proliferation, and survival of cancer cells. KRAS mutation may cause abnormal activation of GTP rather than hydrolysis of GTP into GDP, leading to continuous cell growth signaling and thus abnormal proliferation, eventually causing cancer (Andrew M. Waters et al., Cold Spring Harb Perspect Med. 8(9), a031435, 2018).

Overexpression of KRAS protein is found mainly in pancreatic cancer patients among solid cancers, and most frequent KRAS gene mutations are generally mutations at codon 12, and most frequent KRAS gene mutations in pancreatic cancer are G12D and G12V mutations (Kirsten L. et. al., Trends Biochem Sci. 39(2), 91-100, 2014; Louis Buscail et. al., Nature Rev Gastroenterol Hepatol 17, 153-168, 2020).

Meanwhile, unlike traditional drugs, nucleic acid drugs inhibit the expression of target-specific messenger RNA (mRNA) and thus research is ongoing into the use of nucleic acid drugs in areas in which treatment with conventional drugs that target proteins is impossible. Various clinical trials using nucleic acids are in progress due to performance and advantages thereof as drugs and the use of carriers for intracellular introduction or blood-brain barrier penetration is extremely limited despite the increasing use of nucleic acid-based therapeutics.

Recently, in order to promote safer and more effective use of drugs, not only formulations suitable for drugs but also methods of efficiently delivering drugs to target sites in the body are receiving attention. In particular, there is demand for practical application of a drug delivery system (DDS) that efficiently transports a drug in a required amount to a required place when needed.

In this regard, the present inventors have found that a nucleic acid complex in which a bioactive nucleic acid complementarily binds to a carrier peptide nucleic acid modified to have a net positive charge has surprisingly improved cell permeability, and this enables the expression of the target gene to be regulated very efficiently, and filed a patent application on a novel construct with low cytotoxicity and improved cell permeability and ability to regulate gene expression of bioactive nucleic acids (PCT/KR2017/008636). In addition, the present inventors have continuously conducted research on the function of the construct and developed a substance that aids in endosomal escape to significantly improve the intracellular activity of bioactive nucleic acids (KR 10-2019-0096148).

Accordingly, as a result of intensive efforts to apply nucleic acid complexes having excellent cell permeability to the treatment of pancreatic cancer, the present inventors have found that a nucleic acid complex in which a bioactive peptide nucleic acid targeting the KRAS gene complementarily binds to a carrier peptide nucleic acid is highly effective in preventing or treating pancreatic cancer and thus completed the present invention.

**Summary of the Invention**

It is an object of the present invention to provide a pharmaceutical composition for preventing or treating pancreatic cancer comprising a nucleic acid complex as an active ingredient.

In order to accomplish the above objects, the present invention provides a pharmaceutical composition for preventing, ameliorating or treating pancreatic cancer comprising, as an active ingredient, a cell-permeable nucleic acid complex in which a carrier peptide nucleic acid complementarily binds to a bioactive nucleic acid targeting a KRAS gene.

In addition, the present invention provides a method of treating or preventing pancreatic cancer comprising administering a cell-permeable nucleic acid complex in which a carrier peptide nucleic acid complementarily binds to a bioactive nucleic acid targeting a KRAS gene.

In addition, the present invention provides the use of the cell-permeable nucleic acid complex in which a carrier peptide nucleic acid complementarily binds to a bioactive nucleic acid targeting a KRAS gene for the prevention or treatment of pancreatic cancer.

In addition, the present invention provides the use of the cell-permeable nucleic acid complex in which a carrier peptide nucleic acid complementarily binds to a bioactive nucleic acid targeting a KRAS gene for the manufacture of a medicament for preventing or treating pancreatic cancer.

**Brief Description of Drawings**

FIG. 1A shows the cell viability for each combination after treating human pancreatic cancer cells (Bxpc-3) with the peptide nucleic acid complex of the present invention.

FIG. 1B show the cell viability for each combination after treating human pancreatic cancer cells (PANC-1) expressing mutant KRAS with the peptide-nucleic acid complex of the present invention.

FIG. 2A shows the expression of KRAS-targeting genes and downstream genes for each combination after treating human pancreatic cancer cells (Bxpc-3) with the peptide nucleic acid complex of the present invention.

FIG. 2B shows the expression of KRAS-targeting genes and downstream genes for each combination after treating human pancreatic cancer cells (PANC-1) expressing mutant KRAS with the peptide-nucleic acid complex of the present invention.

FIG. 3 shows the tumor size (A), tumor shape (B) and tumor weight (C) over time after treating an animal model xenografted with human pancreatic cancer cells (Bxpc-3) with the peptide nucleic acid complex of the present invention.

FIG. 4 shows the expression of KRAS-targeting genes and downstream genes in a tumor sample obtained after treating an animal model xenografted with human pancreatic cancer cells (Bxpc-3) with the peptide nucleic acid complex of the present invention.

FIG. 5 shows the result of H&E staining for identifying the phenotype of the tumor tissue obtained after treating an animal model xenografted with human pancreatic cancer cells (Bxpc-3) with the peptide nucleic acid complex of the present invention.

FIG. 6 shows the result of IHC for identifying an expression change of KRAS-targeting genes in the tumor tissue obtained after treating an animal model xenografted with human pancreatic cancer cells (Bxpc-3) with the peptide nucleic acid complex of the present invention.

FIG. 7 shows apoptosis of tumor tissue obtained after treating an animal model xenografted with human pancreatic cancer cells (Bxpc-3) with the peptide nucleic acid complex of the present invention.

FIG. 8 shows the tumor size (A), tumor shape (B) and tumor weight (C) over time after treating an animal model xenografted with human pancreatic cancer cells (PANC-1) expressing mutant KRAS with the peptide nucleic acid complex of the present invention.

FIG. 9A shows the expression of KRAS-targeting genes and downstream genes in tumor samples obtained after treating an animal model xenografted with human pancreatic cancer cells (PANC-1) expressing mutant KRAS with 1 mg/kg of the peptide nucleic acid complex of the present invention.

FIG. 9B shows the expression of KRAS-targeting genes and downstream genes in tumor samples obtained after treating an animal model xenografted with human pancreatic cancer cells (PANC-1) expressing mutant KRAS with 2 mg/kg of the peptide nucleic acid complex of the present invention.

FIG. 10A shows the result of H&E staining identifying the phenotype of the tumor tissue obtained after treating an animal model xenografted with human pancreatic cancer cells (PANC-1) expressing mutant KRAS with 1 mg/kg of the peptide nucleic acid complex of the present invention.

FIG. 10B shows the result of H&E staining identifying the phenotype of the tumor tissue obtained after treating an animal model xenografted with human pancreatic cancer cells (PANC-1) expressing mutant KRAS with 2 mg/kg of the peptide nucleic acid complex of the present invention.

FIG. 11A shows the result of IHC for identifying an expression change of KRAS-targeting genes in the tumor tissue obtained after treating an animal model xenografted with human pancreatic cancer cells (PANC-1) expressing mutant KRAS with 1 mg/kg of the peptide nucleic acid complex of the present invention.

FIG. 11B shows the result of IHC for identifying an expression change of KRAS-targeting genes in the tumor tissue obtained after treating an animal model xenografted with human pancreatic cancer cells (PANC-1) expressing mutant KRAS with 2 mg/kg of the peptide nucleic acid complex of the present invention.

FIG. 12A shows apoptosis of tumor tissue obtained after treating an animal model xenografted with human pancreatic cancer cells (PANC-1) expressing mutant KRAS with 1 mg/kg of the peptide nucleic acid complex of the present invention.

FIG. 12B shows apoptosis of tumor tissue obtained after treating an animal model xenografted with human pancreatic cancer cells (PANC-1) expressing mutant KRAS with 2 mg/kg of the peptide nucleic acid complex of the present invention.

FIG. 13A shows the cell viability for each combination after treating human pancreatic cancer cells (PANC-1) expressing mutant KRAS (G12D) with the peptide nucleic acid complex of the present invention.

FIG. 13B shows the cell viability for each combination after treating human pancreatic cancer cells (CFPAC-1) expressing mutant KRAS (G12V) with the peptide nucleic acid complex of the present invention.

FIG. 14A shows the expression of KRAS-targeting genes and downstream genes after treating human pancreatic cancer cells (PANC-1) expressing mutant KRAS (G12D) with the peptide nucleic acid complex of the present invention.

FIG. 14B shows the expression of KRAS-targeting genes and downstream genes after treating human pancreatic cancer cells (CFPAC-1) expressing mutant KRAS (G12V) with the peptide nucleic acid complex of the present invention.

## Detailed Description and Preferred Embodiments of the Invention

Unless defined otherwise, all technical and scientific terms used herein have the same meanings as appreciated by those skilled in the field to which the present invention pertains. In general, the nomenclature used herein is well-known in the art and is ordinarily used.

The present inventors aimed to determine whether or not administration of a nucleic acid complex in which a bioactive peptide nucleic acid targeting a KRAS gene complementarily binds to a carrier peptide nucleic acid is highly effective in preventing or treating pancreatic cancer.

That is, in one embodiment of the present invention, administration, to pancreatic cancer cell lines and pancreatic cancer cell xenograft models, of a nucleic acid complex, in which a bioactive peptide nucleic acid having a sequence binding to a KRAS gene, KRAS G12D mutant gene, or KRAS G12V mutant gene complementarily binds to a carrier peptide nucleic acid, causes efficient inhibition of the expression of the KRAS genes and downstream genes thereof and thus effectively treats pancreatic cancer.

Accordingly, in one aspect, the present invention is directed to a pharmaceutical composition for preventing, ameliorating or treating pancreatic cancer comprising, as an active ingredient, a nucleic acid complex in which a carrier peptide nucleic acid complementarily binds to a bioactive nucleic acid targeting a KRAS gene.

In the present invention, the bioactive nucleic acid may be bioactive peptide nucleic acid.

In the present invention, the nucleic acid complex in which the bioactive peptide nucleic acid complementarily binds to the carrier peptide may have a structure represented by the following Formula (1):

## **Formula (1)**

## $$[A \equiv C^{(+)}]$$

Wherein

A is a bioactive nucleic acid having a sequence capable of binding to a target gene or a target gene sequence,
C is a carrier peptide nucleic acid capable of binding to the bioactive nucleic acid, and
'$\equiv$' means complementary binding between the bioactive nucleic acid and the carrier peptide nucleic acid,
wherein the bioactive nucleic acid represented by A is a peptide nucleic acid which has a net negative charge,
the carrier peptide nucleic acid represented by $C^{(+)}$ has a net positive charge,
the nucleic acid complex of Formula (1) represented by $A \equiv C^{(+)}$ has a net positive charge, and
the carrier peptide nucleic acid comprises at least one gamma- or alpha-backbone-modified peptide nucleic acid monomer such that the carrier peptide nucleic acid has an net positive charge, and the gamma- or alpha-backbone-modified peptide nucleic acid monomer comprises monomers having positively charged amino acids, the number of which is higher than the number of monomers having negatively charged amino acids, so the carrier peptide nucleic acid has a net positive charge.

As used herein, the term "bioactive nucleic acid" refers to a nucleic acid having a complementary sequence capable of binding to a gene that is the target of expression inhibition, in particular, a nucleic acid having a complementary sequence capable of binding to the mRNA of the gene that is the target of expression inhibition, or a nucleic acid having a sequence that is the target of the expression promotion, means a nucleic acid involved in the regulation of gene expression, such as inhibiting or promoting the expression of the corresponding gene, or a nucleic acid having a sequence complementary to a gene that is the target of expression inhibition or promotion, or a nucleic acid having a sequence complementary to a single-stranded RNA sequence such as pre-mRNA, miRNA, and mRNA.

In particular, the "bioactive peptide nucleic acid" used herein binds to a target gene and a nucleotide sequence comprising the same *in vitro* or *in vivo* and thus acts to activate or inhibit the inherent functions (transcript expression or protein expression) of the corresponding gene, or to regulate splicing of pre-mRNA (e.g., exon skipping), and the base sequence is a gene regulatory sequence, gene coding sequence or a splicing regulatory sequence. The gene regulatory region is a promoter, a transcriptional enhancer, a 5' untranslated region, a 3' untranslated region, a viral packaging sequence, and a selectable marker. The gene region may be an exon or an intron, and the gene region may be present within 10, 5, 3, or 1 kb or 500, 300, or 200 bp of the transcription initiation site of the gene, for example, upstream or downstream of the initiation site. In addition, the splicing regulatory region may comprise sequences related to exon skipping, cryptic splicing, pseudo-splice site activation, intron retention, and alternative splicing deregulation.

Therefore, the "bioactive nucleic acid" in the present invention is preferably an antisense peptide nucleic acid of KRAS, which is a target gene for pancreatic cancer, and comprises more preferably any one of sequences represented by SEQ ID NO: 1, 8 or 9, but is not limited thereto.

In the present invention, the bioactive peptide nucleic acid comprising the sequence represented by the sequence of SEQ ID NO: 8 may target the G12D mutation of the KRAS gene, and the bioactive peptide nucleic acid comprising the sequence represented by SEQ ID NO: 9 may target the G12V mutation of the KRAS gene.

As used herein, the term "carrier peptide nucleic acid" refers to a nucleic acid, the bases of which are partly or entirely complementarily bound to the bioactive nucleic acid, to impart functionality thereto, and the carrier peptide nucleic acid used herein may be a peptide nucleic acid (PNA) or a modified nucleic acid similar thereto, and is preferably a peptide nucleic acid, but is not limited thereto.

In particular, the carrier peptide nucleic acid preferably comprises a sequence selected from the group consisting of sequences represented by SEQ ID NOS: 3 to 6 and 11 to 18, but is not limited thereto.

As used herein, the nucleic acid complex is capable of allowing the bioactive substance to enter the body, ultimately cells, and specifically has cell permeability.

Accordingly, in the present invention, the nucleic acid complex may have cell permeability.

In the present invention, each of the bioactive nucleic acid and the carrier peptide nucleic acid may comprise 2 to 50, preferably 5 to 30, more preferably 10 to 25, most preferably 15 to 17 nucleic acid monomers.

In the present invention, the nucleic acid complex comprises a bioactive nucleic acid comprising a sequence represented by SEQ ID NO: 1, 8 or 9, and a carrier peptide nucleic acid comprising a sequence represented by any one selected from the group consisting of SEQ ID NOS: 3 to 6 and 11 to 18, but is not limited thereto.

The composition of the present invention comprises, as an active ingredient, (i) a nucleic acid complex comprising a bioactive nucleic acid having a sequence represented by SEQ ID NO: 1 and a carrier peptide nucleic acid having a sequence represented by SEQ ID NO: 3;

(ii) a nucleic acid complex comprising a bioactive nucleic acid having a sequence represented by SEQ ID NO: 1 and a carrier peptide nucleic acid having a sequence represented by SEQ ID NO: 4;

(iii) a nucleic acid complex comprising a bioactive nucleic acid having a sequence represented by SEQ ID NO: 1 and a carrier peptide nucleic acid having a sequence represented by SEQ ID NO: 5;

(iv) a nucleic acid complex comprising a bioactive nucleic acid having a sequence represented by SEQ ID NO: 1 and a carrier peptide nucleic acid having a sequence represented by SEQ ID NO: 6;

(v) a nucleic acid complex comprising a bioactive nucleic acid having a sequence represented by SEQ ID NO: 8 and a carrier peptide nucleic acid having a sequence represented by SEQ ID NO: 11;

(vi) a nucleic acid complex comprising a bioactive nucleic acid having a sequence represented by SEQ ID NO: 8 and a carrier peptide nucleic acid having a sequence represented by SEQ ID NO: 12;

(vii) a nucleic acid complex comprising a bioactive nucleic acid having a sequence represented by SEQ ID NO: 8 and a carrier peptide nucleic acid having a sequence represented by SEQ ID NO: 13;

(viii) a nucleic acid complex comprising a bioactive nucleic acid having a sequence represented by SEQ ID NO: 8 and a carrier peptide nucleic acid having a sequence represented by SEQ ID NO: 14;

(ix) a nucleic acid complex comprising a bioactive nucleic acid having a sequence represented by SEQ ID NO: 9 and a carrier peptide nucleic acid having a sequence represented by SEQ ID NO: 15;

(x) a nucleic acid complex comprising a bioactive nucleic acid having a sequence represented by SEQ ID NO: 9 and a carrier peptide nucleic acid having a sequence represented by SEQ ID NO: 16;

(xi) a nucleic acid complex comprising a bioactive nucleic acid having a sequence represented by SEQ ID NO: 9 and a carrier peptide nucleic acid having a sequence represented by SEQ ID NO: 17; and

(xii) a nucleic acid complex comprising a bioactive nucleic acid having a sequence represented by SEQ ID NO: 9 and a carrier peptide nucleic acid having a sequence represented by SEQ ID NO: 18.

In the present invention, the bioactive nucleic acid or the carrier peptide nucleic acid may further comprise a material for facilitating endosomal escape that is additionally bound to the 5'-end or 3'-end of each nucleic acid. That is, the bioactive nucleic acid or the carrier peptide nucleic acid may further comprise a material for facilitating endosomal escape of the bioactive nucleic acid and the carrier peptide nucleic acid and thus have the structure represented by the following Formula (2).

## Formula (2)

$$[\mathrm{mA} \equiv \mathrm{mC}^{(+)}]$$

Wherein

"m" represents a material for facilitating endosomal escape of the bioactive nucleic acid and the carrier peptide nucleic acid.

In the present invention, the "material for facilitating endosomal escape" may facilitate escape of the bioactive nucleic acid from the endosomes by increasing the osmotic pressure in the endosomes or destabilizing the membrane of the endosomes, which means that the "material that facilitates endosomal escape" enables the bioactive nucleic acid to move more efficiently and rapidly to the nucleus or cytoplasm and to meet and function with the target gene (D. W. Pack, A. S. Hoffman, S. Pun, P. S. Stayton, "Design and development of polymers for gene delivery," Nat. Rev. Drug.

Discov., 4, 581-593 (2005)).

In the present invention, the material for facilitating endosomal escape may be at least one selected from the group consisting of peptides, lipid nanoparticles, polyplex nanoparticles, polymer nanospheres, inorganic nanoparticles, cationic lipid-based nanoparticles, cationic polymers, and pH-sensitive polymers.

In the present invention, as the material for facilitating endosomal escape, a peptide having a sequence of GLFDIIKKIAESF (SEQ ID NO: 20) may be bound to the bioactive nucleic acid via a linker, and histidine(10) may be bound to the carrier peptide nucleic acid via a linker, but the present invention is not limited thereto.

In the present invention, the lipid nanoparticles may be selected from the group consisting of lipids, phospholipids, acetyl palmitate, poloxamer 18, Tween 85, tristearin glyceride, and Tween 80.

In the present invention, the polyplex nanoparticles may be poly(amidoamine) or polyethylenimine (PEI).

In the present invention, the polymer nanospheres may be selected from the group consisting of polycaprolactone, poly(lactide-co-glycolide), polylactide, polyglycolide, poly(d,l-lactide), chitosan, and PLGA-polyethylene glycol.

In the present invention, the inorganic nanoparticles may be selected from the group consisting of $Fe_2O_3$, $Fe_3O_4$, $WO_3$ and $WO_{2.9}$.

In the present invention, the cationic lipid-based nanoparticles may be selected from the group consisting of 1-(aminoethyl)iminobis[N-(oleicylcysteinyl-1-aminoethyl)propionamide], an N-alkylated derivative of PTA, and 3,5-didodecyloxybenzamidine.

In the present invention, the cationic polymer may be selected from the group consisting of vinylpyrrolidone-N,N-dimethylaminoethyl methacrylate acid copolymer diethyl sulfate, polyisobutylene, and poly(N-vinylcarbazole).

In the present invention, the pH-sensitive polymers may be selected from the group consisting of polyacids, poly(acrylic acid), poly (methacrylic acid) and hydrolyzed polyacrylamide.

In the present invention, the bioactive nucleic acid may consist of a natural nucleic acid base and/or a modified nucleic acid monomer. The carrier peptide nucleic acid may have a sequence partially or entirely complementary to the base sequence of the bioactive nucleic acid.

In particular, the carrier peptide nucleic acid may comprise one or more universal bases and all of the carrier peptide nucleic acids may comprise universal bases.

In the present invention, the bioactive nucleic acid is selected from the group consisting of DNA, RNA, or modified nucleic acids including peptide nucleic acid (PNA), phosphorodiamidate morpholino oligonucleotide (PMO), locked nucleic acid (LNA), glycol nucleic acid (GNA), and threose nucleic acid (TNA), antisense oligonucleotide, aptamers, small interfering RNA (siRNA), short hairpin RNA (shRNA), ribozyme, and DNAzyme. Preferably, the bioactive nucleic acid is selected from the group consisting of DNA, RNA, or modified nucleic acids including peptide nucleic acid (PNA), phosphorodiamidate morpholino oligonucleotide (PMO), locked nucleic acid (LNA), glycol nucleic acid (GNA), and threose nucleic acid (TNA).

In the present invention, when the monomer used for the bioactive nucleic acid is PNA, it is called a bioactive peptide nucleic acid, and when other monomers are used, they are referred to in the same manner.

In the present invention, the bioactive nucleic acid and the carrier peptide nucleic acid may further comprise at least one functional group selected from the group consisting of phosphodiester, 2'-0-methyl, 2'-methoxy-ethyl, phosphoramidate, methylphosphonate, and phosphorothioate.

In the present invention, the carrier peptide nucleic acid may have a nucleotide sequence which is partially or entirely complementary to the bioactive nucleic acid. In particular, the carrier peptide nucleic acid may comprise at least one universal base, and the carrier peptide nucleic acid may be entirely composed of universal bases.

In the present invention, with regard to the electrical properties, each of the bioactive nucleic acid and the carrier peptide nucleic acid in the nucleic acid complex may have a net positive charge (positive), a net negative charge (negative), or neutral (no charge).

The term "net" in the expression of the electrical properties means overall electrical properties of respective charges of the bioactive nucleic acids or carrier peptide nucleic acids as viewed from the outside, not the electrical properties of individual bases. For example, even though some monomers in the bioactive nucleic acid are positive, when the number of negatively charged monomers is greater than the number of positively charged monomers, the bioactive nucleic acid is negatively charged in terms of the "net" electrical properties. When the number of positively charged bases and/or backbones is greater than the number of negatively charged bases and/or backbones, even though some bases and/or backbone constituents in the carrier peptide nucleic acid are negatively charged, the carrier peptide nucleic acid is considered to be positively charged in terms of the "net" electrical properties thereof.

Accordingly, the nucleic acid complex of the present invention may be considered to have a net positive charge. In the nucleic acid complex, preferably, the bioactive nucleic acid has a net negative charge or is neutral in terms of overall electrical properties, and the carrier peptide nucleic acid has a net positive charge in terms of overall electrical properties, but is not limited thereto.

In the present invention, a modified peptide nucleic acid monomer may be used to impart electrical properties to the bioactive nucleic acid and the carrier peptide nucleic acid, and the modified peptide nucleic acid monomer comprises, as a positively charged carrier peptide nucleic acid, at least one positively charged nucleic acid selected from the group consisting of lysine (Lys, K), arginine (Arg, R), histidine (His, H), diamino butyric acid (DAB), ornithine (Orn), and amino acid analogs, and comprises, as a negatively charged carrier peptide nucleic acid, glutamic acid (Glu, E), which is a negatively charged amino acid, or an amino acid analogue, which is a negatively charged amino acid.

In the present invention, the carrier peptide nucleic acid may comprise at least one gamma- or alpha-backbone-modified peptide nucleic acid monomer in order for the carrier peptide nucleic acid to have a net positive charge.

The gamma- or alpha-backbone-modified peptide nucleic acid monomer comprises, in the backbone thereof, at least one positively charged amino acid selected from the group consisting of lysine (Lys, K), arginine (Arg, R), histidine (His, H), diamino butyric acid, ornithine (Orn), and amino acid analogs in order for the carrier peptide nucleic acid to have a net positive charge.

In the present invention, the peptide nucleic acid monomer having a modified nucleobase, in addition to the backbone modification, for modification of the peptide nucleic acid monomer so as to impart an electrical charge thereto may be used. Preferably, an amine, triazole or imidazole moiety may be comprised in the nucleobase to impart a positive charge thereto, or carboxylic acid may be comprised in the base to impart a negative charge thereto.

In the present invention, the modified peptide nucleic acid monomer of the carrier peptide nucleic acid may further comprise a negative charge in the backbone or nucleobase. However, preferably, the modified peptide nucleic acid monomer comprises more positively charged monomers than negatively charged monomers, such that the carrier peptide nucleic acid is positively charged.

In the nucleic acid complex according to the present invention, at least one material selected from the group consisting of a hydrophobic moiety, a hydrophilic moiety, a target-antigen-specific antibody, an aptamer, and a fluorescent/luminescent marker is bound to the bioactive nucleic acid and/or the carrier peptide nucleic acid. Preferably, at least one material selected from the group consisting of a hydrophobic moiety, a hydrophilic moiety, a target-antigen-specific antibody, an aptamer and a fluorescent/luminescent marker for imaging may be bound to the carrier peptide nucleic acid.

In the present invention, the binding of at least one material selected from the group consisting of a hydrophobic moiety, a hydrophilic moiety, a target-antigen-specific antibody, an aptamer, a quencher, a fluorescent marker and a luminescent marker to the bioactive nucleic acid and/or the carrier peptide nucleic acid may be a simple covalent bond or a covalent bond mediated by a linker, but is not limited thereto. Preferably, cell permeation, solubility, stability, transportation and imaging-related substances (e.g., hydrophobic residues and the like) bound to the nucleic acid carrier exist independently of the bioactive nucleic acid that regulates the expression of the target gene.

In the present invention, as described above, the complementary binding of the bioactive nucleic acid and the carrier peptide nucleic acid is generally antiparallel binding or parallel binding. Complementary binding forms a structure that is separated in the presence of the target sequence of the bioactive nucleic acid (a sequence complementary to the bioactive nucleic acid).

The antiparallel binding and parallel binding are determined depending on the 5'-direction and the 3'-direction in the binding mode of DNA-DNA or DNA-PNA. Antiparallel binding is a general binding mode of DNA-DNA or DNA-PNA. For example, in the nucleic acid complex according to the present invention, the bioactive nucleic acid in a 5' to 3' direction is bound to the carrier peptide nucleic acid in a 3' to 5' direction. Parallel binding has slightly lower binding force than antiparallel binding, and the bioactive nucleic acid and the carrier peptide nucleic acid are bound to each other in the same direction, that is, the 5' to 3' direction or the 3' to 5' direction.

In the nucleic acid complex according to the present invention, preferably, the binding force between the bioactive nucleic acid and the carrier peptide nucleic acid is lower than the binding force between the bioactive nucleic acid and the target gene targeted by the bioactive nucleic acid, particularly the mRNA of the target gene. The bonding force is determined by the melting temperature (Tm).

Examples of specific methods for lowering the binding force (melting temperature, Tm) between the bioactive nucleic acid and the carrier peptide nucleic acid, compared to the binding force between the bioactive nucleic acid and the target gene targeted by the bioactive nucleic acid, particularly the mRNA of the target gene, comprise parallel binding or partial specific binding between the bioactive nucleic acid and the carrier peptide nucleic acid, but are not limited thereto.

As another example, the carrier peptide nucleic acid comprises at least one peptide nucleic acid base selected from the group consisting of a linker, a universal base, and a peptide nucleic acid base comprising a base that is not complementary to a corresponding base of the bioactive nucleic acid, but is not limited thereto.

In the present invention, the universal base is a base that is non-selectively bound to a natural base such as adenine, guanine, cytosine, thymine or uracil, and has a binding force lower than the complementary binding force, and may be at least one selected from the group consisting of inosine PNA, indole PNA, nitroindole PNA, and abasic PNA, and is preferably inosine PNA.

The present invention provides a combination of the binding mode and electrical properties of nucleic acids for controlling the function of the nucleic acid complex, and controls the particle size and the action time using the combination of the binding mode and electrical properties of the nucleic acids, and improves cell permeability, solubility and specificity.

In the present invention, the time point at which the bioactive peptide nucleic acid binds to the target sequence (such as the time point at which the bioactive nucleic acid is substituted with the target sequence, and the time point at which target-specific release and binding occur) may be controlled in the presence of the target gene through control of the binding force between the bioactive peptide nucleic acid and the carrier peptide nucleic acid.

In the nucleic acid complex according to the present invention, the control of the time point of substitution (strand displacement) of the bioactive nucleic acid with the target gene and the time point of target-specific release and binding is made possible by the presence, number, and position of non-specific bases, universal bases and linkers of carrier nucleic acids for non-specific binding of the complex. The control is possible due to the combination of the factors described above and parallel or antiparallel binding, which is the complementary binding mode of the peptide complex.

As used herein, the terms "disease" and "disorder" are used interchangeably and refers to any change in the state of the body or in some organs which impede and/or disrupt the performance of a function, cause symptoms such as discomfort and dysfunction, or lead to the death of people with a disease or the death of people who come into contact therewith.

In the present invention, the term "therapeutic composition" may be used interchangeably with "pharmaceutical composition", and refers to a composition that comprises, as an active ingredient, a nucleic acid complex comprising the bioactive nucleic acid and the carrier peptide nucleic acid bound to the nucleic acid according to the present invention, and may further comprise a therapeutic drug for treating a desired disease, bound to the nucleic acid complex.

The therapeutic composition of the present invention may be formulated in a form that can be delivered through the blood-brain barrier according to standard pharmaceutical practice. In addition to the active ingredient, these formulations may comprise additives such as carriers, excipients, adjuvants or diluents suitable for pharmaceutically acceptable formulations.

The term "physiologically acceptable" refers to a property of not impairing the biological activity and physical properties of a compound.

The term "carrier" is defined as a compound that facilitates the transport of the nucleic acid complex into cells or tissues. For example, dimethylsulfoxide (DMSO) is a commonly used carrier that facilitates the incorporation of many organic compounds into cells or tissues of an organism.

The term "diluent" is defined as a compound that stabilizes a biologically active form of a target compound and is diluted in water that dissolves the target compound. Salts dissolved in buffer solutions are used as diluents in the art. A commonly used buffer solution is phosphate-buffered saline because it mimics the salinity of human bodily fluids. Because buffer salts can control the pH of a solution at low concentrations, buffer diluents rarely alter the biological activity of compounds.

The substance comprising the nucleic acid complex in the present invention may be administered to a patient alone or as a pharmaceutical composition mixed with other active ingredients, or with suitable carriers or excipients, that is, in combination therapy.

The pharmaceutical composition suitable for use in the present invention comprises compositions comprising the active ingredients in an amount effective to achieve the intended purpose thereof. More specifically, a therapeutically effective amount means an amount of a compound effective to lengthen the survival of the subject to be treated, or to prevent, alleviate or relieve the symptoms of diseases. The determination of the therapeutically effective amount is possible by those skilled in the art, particularly in consideration of the detailed description provided herein.

The term "prevention" as used herein means any action of preventing the onset of a disease or inhibiting the progression thereof by administration of the therapeutic composition comprising the nucleic acid complex.

The term "alleviation" as used herein refers to any action of at least reducing the degree of parameters related to the condition to be treated, for example, the severity of symptoms, by administration of the therapeutic composition comprising the nucleic acid complex.

In addition, the term "treatment" as used herein refers to any action in which symptoms of a disease are alleviated or eliminated by administration of the therapeutic composition comprising the nucleic acid complex.

The composition comprising the nucleic acid complex according to the present invention may be applied in a pharmaceutically effective amount to treat pancreatic cancer or to inhibit (or alleviate) the symptoms of pancreatic cancer. The pharmaceutically effective amount may vary depending on various factors such as the type of pancreatic cancer, the age and weight of the patient, the characteristics and extent of symptoms, the type of current therapy, the number of treatments that are performed, and the application form and route, and can be easily determined by experts in the field. The composition of the present invention may be applied simultaneously or sequentially in combination with the pharmacological or physiological components described above, and may be applied sequentially or simultaneously in combination with additional conventional therapeutic agents. Administration may be performed in one or multiple applications.

As used herein, the term "subject" refers to a mammal, preferably a human that suffers from or is at risk of a condition or disease that can be alleviated, suppressed or treated by administering the nucleic acid complex according to the present invention thereto.

In addition, the amount of the compound of the present invention that is administered to the human body may vary depending on the age, weight and gender of the patient, the administration form, the health status, and the severity of disease, and is generally 0.001 to 1,000 mg/day, preferably 0.01 to 500 mg/day, based on an adult patient weighing 70 kg, and may be administered once a day or in multiple doses (in a portionwise manner) several times a day at regular time intervals according to the prescription of doctors or pharmacists.

The toxicity and therapeutic efficiency of the compositions comprising the nucleic acid complex described herein are, for example, estimated through standard pharmaceutical procedures in cell culture or laboratory animals to determine the LD50 (lethal dose for 50% of the population), ED50 (dose providing a therapeutic effect on 50% of the population) and IC50 (dose providing therapeutic and inhibitory effects on 50% of the population). The ratio of toxicity to therapeutic effect for a dose is called the therapeutic index, and may be expressed as the ratio of LD50 to ED50 (or IC50). Compounds having a high therapeutic index are preferred. The data obtained from these cell culture assays may be used to estimate the range of dose for human applications. The amount (dosage) of such a compound that is administered is preferably within a range of a circulating concentration including ED50 (or IC50) with little or no toxicity.

As used herein, the term "administration" refers to an act of introducing the pharmaceutical composition of the present invention into a subject by any suitable method, and the administration may be performed through any of various routes, either oral or parenteral, as long as it enables the composition to reach the target tissue.

The pharmaceutical composition of the present invention may be administered in a pharmaceutically effective amount. The term "pharmaceutically effective amount" used herein means a sufficient amount used to treat or prevent a disease at a reasonable benefit/risk ratio applicable to medical treatment or prevention. The effective amount is determined depending on factors including the severity of the disease, the activity of the drug, the age, weight, health and gender of the patient, the sensitivity of the patient to the drug, the time of administration, the route of administration, and the rate of excretion and treatment period of the composition of the present invention used, drugs used in combination with or concurrently with the composition of the present invention, and other factors well known in the pharmaceutical field.

The pharmaceutical composition of the present invention may be administered as a single therapeutic agent or in combination with other therapeutic agents, either sequentially or simultaneously. The pharmaceutical composition of the present invention may be administered in single or multiple doses. Taking into consideration these factors, it is important to administer the composition in the minimum amount sufficient to achieve maximum efficacy without side effects.

In addition, the dosage (administered amount) of the pharmaceutical composition according to the present invention may be determined by those skilled in the art in consideration of the purpose of use, the severity of disease, the patient's age, weight, gender, and history, or the substances used as active ingredients. For example, the pharmaceutical composition may be administered to an adult in a daily dose of 10 mg/kg to 100 mg/kg, more preferably 1 mg/kg to 30 mg/kg. The frequency of administration of the composition of the present invention is not particularly limited, and the composition may be administered one to three times a day, or may be divided into multiple doses and administered throughout the day.

In another aspect, the present invention is directed to a method of preventing or treating pancreatic cancer comprising administering, to a subject, a nucleic acid complex in which a bioactive peptide nucleic acid having a sequence capable of binding to a KRAS gene complementarily binds to a carrier peptide nucleic acid, and/or a composition comprising the nucleic acid complex.

In another aspect, the present invention is directed to the use of a nucleic acid complex in which a bioactive peptide nucleic acid having a sequence capable of binding to a KRAS gene complementarily binds to a carrier peptide nucleic acid, and/or a composition comprising the nucleic acid complex for the manufacture of a medicament for preventing or treating of pancreatic cancer.

In another aspect, the present invention is directed to the use of a nucleic acid complex in which a bioactive peptide nucleic acid having a sequence capable of binding to a KRAS gene complementarily binds to a carrier peptide nucleic acid, and/or a composition comprising the nucleic acid complex for the prevention or treatment of pancreatic cancer.

**[Example]**

Hereinafter, the present invention will be described in more detail with reference to the following examples. However, it will be obvious to those skilled in the art that the following examples are provided only for illustration of the present invention and should not be construed as limiting the scope of the present invention based on the subject matter of the present invention.

KRAS was used as a target gene to verify the effect of the nucleic acid complex of the present invention on pancreatic cancer.

In the present invention, in order to determine the anticancer effects on KRAS wild and mutant pancreatic cancer, anticancer effects were determined using KRAS-wild type pancreatic cancer cell lines (BxPC-3), and G12D (PANC-1) and G12V (CFPAC-1) mutant pancreatic cancer cell lines that are the most common in pancreatic cancer. In order to determine the anticancer effect on pancreatic cancer, antisense peptide nucleic acid (antisense PNA) was used as a bioactive peptide nucleic acid targeting KRAS.

**Analysis of therapeutic effects for KRAS wild-type pancreatic cancer of KRAS-targeting antisense peptide nucleic acids**

**Example 1: Bioactive peptide nucleic acid, carrier peptide nucleic acid, and preparation of complexes using the same**

The bioactive nucleic acid (antisense PNA) used as a control has the sequence represented by SEQ ID NO: 2 and the bioactive peptide nucleic acid (antisense PNA) used to determine the anticancer effect on pancreatic cancer has the sequence represented by SEQ ID NO: 1.

The peptide nucleic acid-based bioactive nucleic acid used to determine the anticancer effect for pancreatic cancer in the KRAS wild-type pancreatic cancer cell line has a 5'-end bonded to the peptide GLFDIIKKIAESF to improve the function of endosomal escape, and the base sequence, monomer modification and structure thereof are shown in Table 1 below, and the carrier peptide nucleic acid has 3' and 5'-endes each bonded to the peptide to improve the function of endosomal escape and has sequences represented by SEQ ID NOS: 3 to 7 (Table 1).

All peptide nucleic acids used herein were synthesized at PANAGENE (Korea) by HPLC purification.

[Table 1]

| Sequences of bioactive nucleic acids and carrier peptide nucleic acids to verify anti-cancer effect against pancreatic cancer | | | |
|---|---|---|---|
| Item | SEQ ID NO: | Base sequence | Monomer modification |
| Bioactive nucleic acid | 1 | 5'-GLFDIIKKIAESF-O-ACT$^{(-)}$TG$^{(+)}$CTT$^{(-)}$CCT$^{(+)}$GTA$^{(-)}$GG-O-K-3' | -+-+- |
| | 2 | 5'-GLFDIIKKIAESF-O-AGT$^{(-)}$CTT$^{(+)}$GAC$^{(-)}$TTA$^{(+)}$TT$^{(-)}$G-O-K-3' | -+-+- |
| Carrier peptide nucleic acid | 3 | 5'-Histidine(10)-O-TGA$^{(+)}$ACGAA$^{(+)}$GGACAT$^{(+)}$CC-O-K-3' | +++ |
| | 4 | 5'-K-O-CC$^{(+)}$TACAGG$^{(+)}$AAGCA$^{(+)}$AGT-O-Histidine(10)-3' | +++ |
| | 5 | 5'-Histidine(10)-O-CA$^{(+)}$TC$^{(+)}$C-O-K-3' | ++ |
| | 6 | 5'-K-O-CC$^{(+)}$TA$^{(+)}$C-O-Histidine(10)-3' | ++ |
| | 7 | 5'-Histidine(10)-O-TCA$^{(+)}$GAACTG$^{(+)}$AATAA$^{(+)}$C-O-K-3' | +++ |

Table 1 above shows the sequence information of the bioactive peptide nucleic acid and carrier peptide nucleic acid used to determine the anticancer effect for pancreatic cancer targeting KRAS and the sequence information of bioactive peptide nucleic acid and carrier peptide nucleic acid used as a control.

In order to impart electrical properties, modification of the monomer was performed to produce a modified peptide nucleic acid backbone designed such that the peptide nucleic acid backbone comprises lysine (Lys, K$^{(+)}$) for a positive electrical charge and the modified peptide nucleic acid backbone comprises glutamic acid (Glu, E$^{(-)}$) for a negative electrical charge.

The respective combinations of bioactive nucleic acids and carrier peptide nucleic acids were hybridized in the presence of DMSO to produce a complex comprising the bioactive nucleic acid and the carrier peptide nucleic acid.

**Example 2: Analysis of therapeutic effect for pancreatic cancer using nucleic acid complexes**

The anticancer effect for pancreatic cancer was analyzed using the nucleic acid complex comprising the carrier peptide nucleic acid and the bioactive peptide nucleic acid targeting KRAS prepared to have the structure of the following Table 2 according to Example 1.

Example 2-1: Cell culture

Human pancreatic cancer cell Bxpc-3 (ATCC NO. 1687) obtained from ATCC (American Type Culture Collection, USA) and human pancreatic cancer cell PANC-1 expressing mutant KRAS (ATCC NO. 1496) were incubated at 37°C in the presence of 5% (v/v) $CO_2$ in RPMI culture medium (Roswell Park Memorial Institute Medium, Wellgene, Korea) and DMEM culture medium (Dulbecco's Modified Eagle's Medium, Wellgene, Korea) supplemented with 10% (v/v) fetal bovine serum, 100 units/ml of penicillin, and 100 ug/ml of streptomycin, respectively.

Example 2-2: Analysis of cell viability of pancreatic cancer cell lines by MTT (MTT assay)

Pancreatic cancer cell lines Bxpc-3 and PANC-1 were seeded at $4 \times 10^3$ in a 96-well plate under the experimental conditions in Example 2-1, cultured for 24 hours, and treated with complexes comprising bioactive nucleic acids and carrier peptide nucleic acids prepared with the structures shown in Table 1, and the resulting cell lines were treated at a concentration of 20 pL/well with a solution of MTT (3-(4,5-dimethylthiazol-2-yl)-2,5-diphenyltetrazolium bromide) at a concentration of 5 mg/mL in 1X PBS and incubated for 4 hours, and OD (optical density) thereof was measured and analyzed with a spectrophotometer.
Nucleic acid complex combinations used in this Example are shown in Table 2 below.

[Table 2]

| Combination of nucleic acid complexes for cell viability analysis in human pancreatic cancer cell lines | |
| --- | --- |
| Combination | Nucleic acid complex |
| 1 | SEQ ID NOS: 1 and 3 |
| 2 | SEQ ID NOS: 1 and 4 |
| 3 | SEQ ID NOS: 1 and 5 |
| 4 | SEQ ID NOS: 1 and 6 |
| 5 | SEQ ID NOS: 2 and 7 (control) |

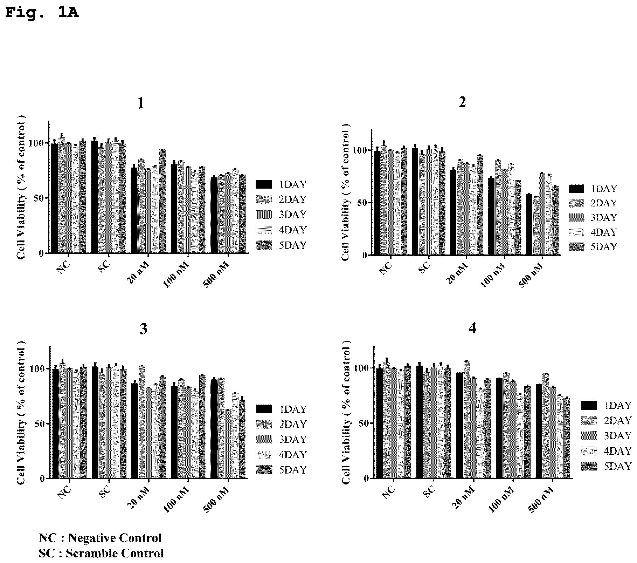

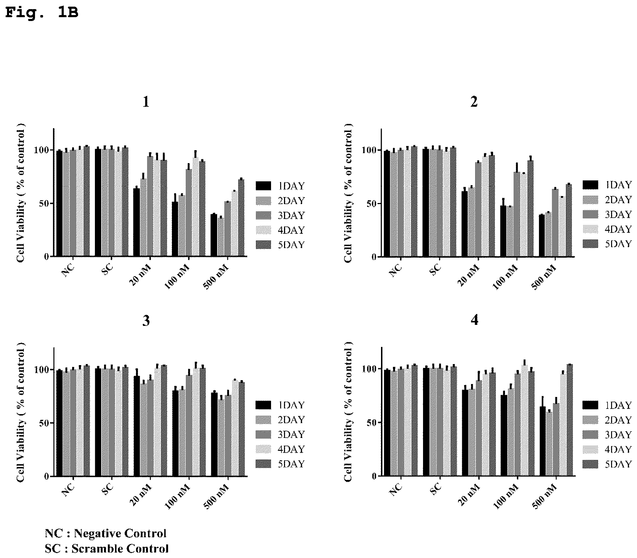

As a result, as can be seen from FIG. 1, the nucleic acid complex Combination No. 1 of Table 2 decreased the cell viability in a concentration-dependent manner for up to 72 hours in the Bxpc-3 human pancreatic cancer cell model (FIG. 1A). In addition, it can be seen that, in human pancreatic cancer cells PANC-1 expressing mutant KRAS, the combination No. 1 nucleic acid complex decreased the cell viability the most for up to 96 hours (FIG. 1B).

Example 2-3: Analysis of gene expression by Western blot assay

The human pancreatic cancer cell lines Bxpc-3 and PANC-1 were seeded at $1 \times 10^5$ in a 6-well plate and cultured for 24 hours, an experiment was performed under the conditions of Example 2-1, the cell lines were treated with a complex comprising bioactive peptide nucleic acid and carrier peptide nucleic acid, incubated for 24, 48, 72, 96, and 120 hours, and 30 $\mu$L of RIPA buffer was added to each well to obtain a protein lysate. The protein was assayed from the protein lysate using a BCA assay kit (Thermo Fisher, USA), 30 $\mu$g of protein was separated by size through electrophoresis, and the protein was transferred to a PVDF membrane, followed by treatment with primary antibodies, KRAS (Abcam, UK), p-ERK (Cell Signaling Technology, USA), HRAS (Abcam, UK), and NRAS (Abcam, UK) at a ratio of 1:1000 and allowing to stand at 4°C for one day.
The product was washed with 1XT BS-T, treated with, as secondary antibodies, goat anti-rabbit (Cell Signaling Technology, USA) and mouse anti-goat (Santa cruz Biotechnology, USA) at a ratio of 1:2,000, and allowed to stand at room temperature for 1 hour. The result was treated with Supersignal™ West Femto Maximum Sensitivity Substrate (Thermo Fisher, USA), and the efficiency of suppression of target gene expression was analyzed in pancreatic cancer cell lines using Imager 600 (Amersham, Germany).

In this example, expression changes of RAS family (KRAS, HRAS, NRAS) and downstream genes in human pancreatic cancer cell models were analyzed, and the nucleic acid complex combinations used herein are shown in Table 2.

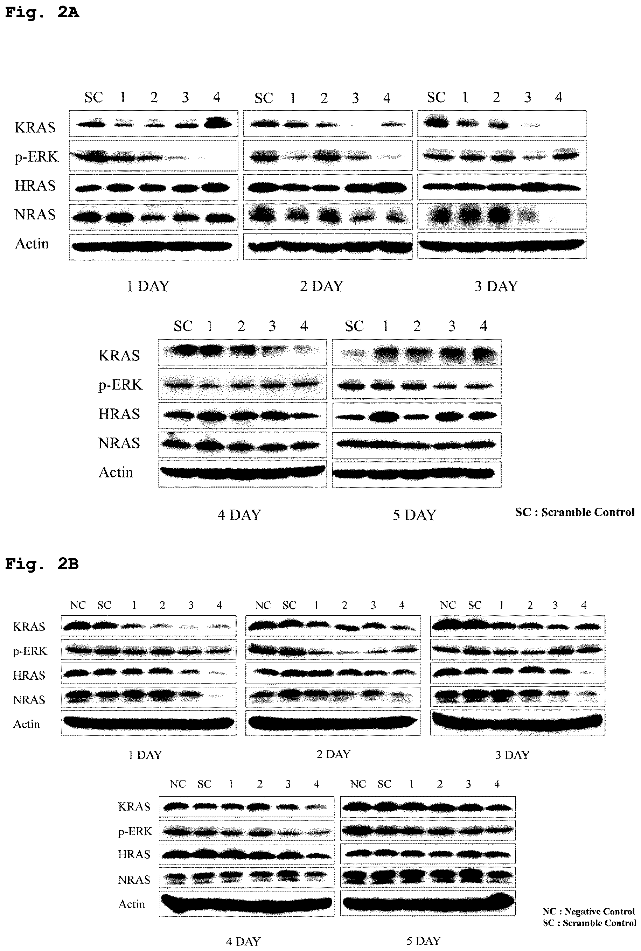

As a result, as can be seen from FIG. 2, the nucleic acid complex of combination of SEQ ID NOS: 1 and 3 inhibited expression of the KRAS-targeting gene in the RAS family and expression of the downstream gene p-ERK for up to 72 hours in Bxpc-3 human pancreatic cancer cells, compared to the nucleic acid complex of combination of SEQ ID NOS: 2 and 7 (FIG. 2A). In addition, in human pancreatic cancer cells PANC-1 expressing mutant KRAS, Combination 1 in Table 2 exhibited the same effect of reducing KRAS and p-ERK up to 72 hours (FIG. 2B). Combinations 2, 3, and 4 reduced HRAS and NRAS among the RAS family and exhibited an off-target effect on undesired sequences, whereas Combination 1 did not exhibit the off-target effect and exhibited significant inhibitory effects on the expression of KRAS genes and downstream genes (FIGS. 2A and 2B).

**Example 3: Pancreatic cancer phenotype effect analysis in xenograft mouse model using Bxpc-3 human pancreatic cancer cells**

5-week-old female nude mice (Orient Bio, Korea) were used to verify efficacy in the xenograft mouse model. To construct a tumor model, Bxpc-3 human pancreatic cancer cells were subcutaneously injected in an amount of $5 \times 10^6/100 \mu$l into the armpit between the right shoulder blade and the chest wall in the subcutaneous fat layer of the dorsal side of the mice. The mice were allowed to stand for 3 weeks for tumor growth and then nucleic acid complexes were administered by tail vein injection to three mice of each experimental group twice a week. As a positive control, Gemcitabine (Sigma, USA), a primary standard anticancer drug for human pancreatic cancer patients, was intraperitoneally administered at a dose of 80 mg/kg to the mice twice a week. The nucleic acid complex and the positive control were administered a total of six times for 3 weeks and then all animals were euthanized.

In this example, nucleic acid complex combinations whose effects were verified in Example 2 were selected and histological findings and KRAS gene expression changes were analyzed in an animal model xenografted with Bxpc-3 human pancreatic cancer cells. The nucleic acid complex combinations used herein are shown in Table 3 below.

[Table 3]

| Combination of nucleic acid complexes for analysis of antitumor effect in xenograft animal model using human pancreatic cancer cells | |
|---|---|
| Combination | Nucleic acid complex |
| 1 | SEQ ID NOS: 1 and 3 |

Example 3-1: Analysis of tumor size change in pancreatic cancer xenograft animal model

In animal experiments conducted under the conditions of Example 3, the length (L) and width (W) of each mouse were measured using vernier calipers (Mitutoyo, Japan) twice a week a total of 6 times from the time at which the average tumor size by group reached 32.3 mm$^3$ after cancer cell transplantation to the 21$^{st}$ day. In addition, at the end of the final experiment, tumors were separated from the back of the mice and the appearances of the tumors were compared to observe differences in size and weight.

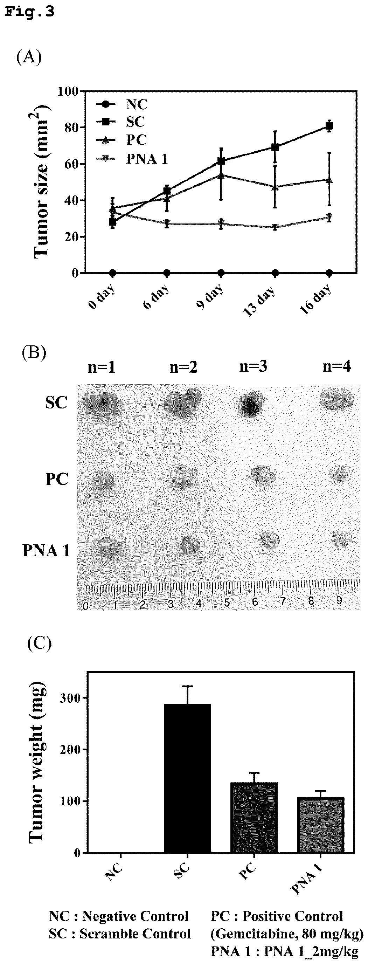

As a result, as can be seen from FIG. 3, compared to the control (scramble control, SC) and positive control (Gemcitabine, PC), the average tumor size in the group treated with the nucleic acid complex of SEQ ID NOS: 1 and 3 (combination 1) decreased over the entire period. In addition, a statistically significant reduction in tumor weight was observed in the group administered the nucleic acid complex (Combination 1) compared to the scramble control and the positive control.

Example 3-2: Analysis of gene expression using Western blot assay

RIPA buffer was added at 200 $\mu$L/well to a part of the biopsied mouse tumor tissue after the experiment under the conditions of Example 3 to obtain a protein lysate. The protein was assayed from the protein lysate using a BCA assay kit (Thermo Fisher, USA), 30 $\mu$g of protein was separated by size through electrophoresis, and the protein was transferred to a PVDF membrane, treated at a ratio of 1:1000 with primary antibodies, namely, KRAS (Abcam, UK), p-ERK (Cell Signaling Technology, USA), HRAS (Abcam, UK), and NRAS (Abcam, UK), and allowed to stand at 4°C for one day.

The result was washed with 1X TBS-T, treated with secondary antibodies, namely, Goat Anti-Rabbit (Cell Signaling Technology, USA) and Mouse Anti-Goat (Santa Cruz Biotechnology, USA) at a ratio of 1:2000 and allowed to stand at room temperature for 1 hour. The result was treated with Supersignal™ West Femto Maximum Sensitivity Substrate (Thermo Fisher, USA) and efficiency of suppression of target gene expression was analyzed in pancreatic cancer tissue using Imager 600 (Amersham, Germany) equipment.

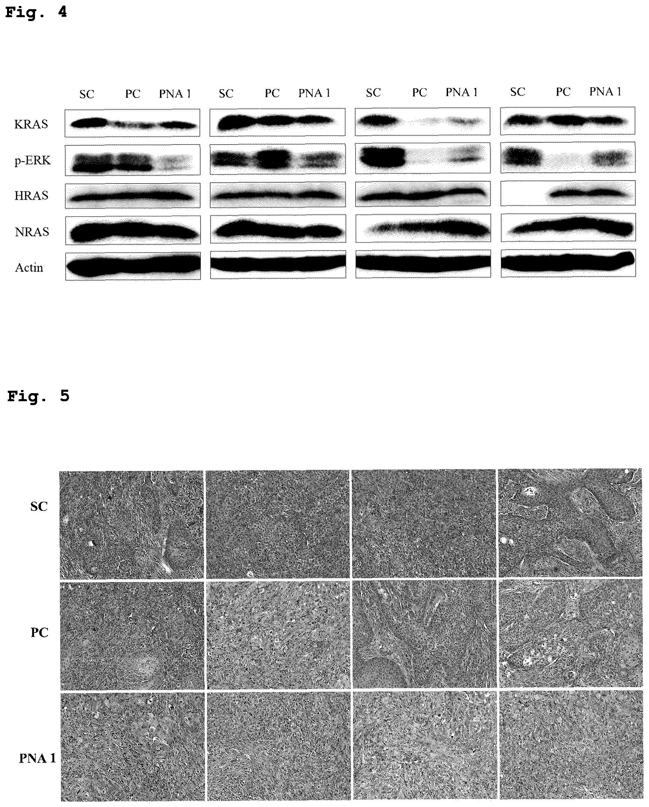

As a result, as can be seen from FIG. 4, the expression of the KRAS-targeting gene in the RAS family and the expression of the downstream gene p-ERK decreased in the group treated with the nucleic acid complex (combination 1) compared to the control. In particular, it was observed that the expression of the p-ERK gene decreased more than that of the positive control.

Example 3-3: Phenotype analysis in pancreatic cancer xenograft animal models by H&E staining

Experiments were conducted under the conditions of Example 3 and tumor tissues were removed from the inoculation site of the cell line on the final day of the experiment. Tissues were fixed in a 4% formalin solution for 24 hours and then paraffin-embedded, and the tissues were sectioned at 5 um and mounted on slide glass. The mounted tissues were stained with Hematoxylin:Eosin staining solution for a predetermined period of time and then washed with 1X PBS, and morphological findings were analyzed under a microscope.

As a result, it was found that the phenotype of the tumor formed as shown in FIG. 5 was reduced in the distribution of inflammatory cells in the group treated with the nucleic acid complex (combination 1) compared to the control and was similar to that of the positive control, which is a cytotoxic anticancer agent.

Example 3-4: Analysis of KRAS expression changes in tissues in pancreatic cancer xenograft animal models by immunostaining

The experiment was conducted under the conditions of Example 3. On the final day of the experiment, the tumor tissue was removed from the inoculation site of the cell line and fixed in 4% formalin solution for one day, and the fixed tissue was paraffin-embedded, sectioned into 5 um sections, and mounted on a slide glass. The mounted tissue was blocked in 0.5% BSA solution for 1 hour and allowed to react with KRAS primary antibody (Abcam, UK) at 4°C for one day. Then, the primary antibody solution was removed, washed with 1X TBS, treated with secondary antibody solution, namely Goat Anti-Rabbit (Cell Signaling Technology, USA) at a ratio of 1:2000 at room temperature for 2 hours, and then analyzed by DAB staining.

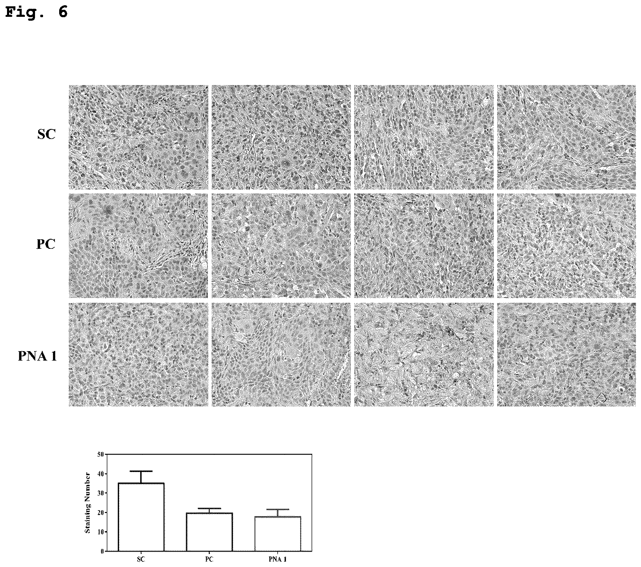

As a result, as can be seen from FIG. 6, brown-stained KRAS was expressed in the control tissue and a decrease in KRAS expression in nucleic acid complex combination 1 was observed in all four tumor tissues. The results were digitized and then compared. The result showed that the reduction in expression by nucleic acid complex combination 1 was similar to that of the positive control.

Example 3-5: Analysis of apoptosis in pancreatic cancer xenograft animal models by TUNEL assay

The experiment was conducted under the conditions of Example 3. On the final day of the experiment, the tumor tissue was removed from the inoculated site of the cell line and fixed in 4% formalin solution for one day, and the fixed tissue was paraffin-embedded, sectioned into 5 um sections, and mounted on a slide glass. A reaction TdT buffer was dropped on the mounted tissue, followed by reaction at 37°C for 70 minutes. Then, the result was reacted with BrdU-Biotin antibody solution in the absence of light for 70 minutes and apoptotic cells were stained with DAB solution. After the reaction was completed, the tissue was immersed in a hematoxylin solution to stain the nuclei, washed with running water for 10 minutes, and mounted, and how much apoptosis occurred was observed under an optical microscope.

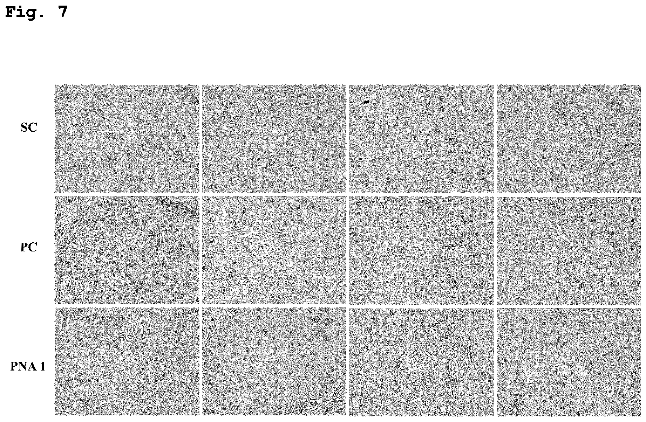

As a result, as can be seen from FIG. 7, more green-stained nuclei than brown-stained apoptotic cells were present in the control tissue and the distribution of apoptotic cells increased in the nucleic acid complex combination 1 and the positive control, which indicates an increase in apoptosis in tumor tissue.

**Example 4: Analysis of pancreatic cancer phenotypic effects in subcutaneously transplanted animal model (xenograft mouse model) using PANC-1 human-derived pancreatic cancer cells**

5-week-old female nude mice (Orient Bio, Korea) were used to verify efficacy in the xenograft animal model. To construct a tumor model, the cell concentration of PANC-1 human-derived pancreatic cancer cells was adjusted to $3 \times 10^7$ /ml using Matrigel. The adjusted cell culture medium was subcutaneously injected at 0.3 ml into the armpit between the right scapula and the chest wall ($9 \times 10^6$/mouse). The mice were allowed to stand for 1 week for tumor growth and then nucleic acid complexes were administered by tail vein injection into the six mice of each experimental group twice a week. As a positive control, Gemcitabine (Sigma, USA), a primary standard anticancer drug for human pancreatic cancer patients, was intraperitoneally administered to the mice at a dose of 80 mg/kg twice a week. The nucleic acid complex and the positive control were administered a total of six times for 3 weeks and then all animals were euthanized.

In this example, nucleic acid complex combinations whose effects were verified in Example 2 were selected and histological findings and KRAS gene expression changes were analyzed in an animal model xenografted with PANC-1 human pancreatic cancer cells. The nucleic acid complex combinations used herein are shown in Table 3 above.

Example 4-1: Analysis of tumor size change in pancreatic cancer xenograft animal model

In animal experiments conducted under the conditions of Example 4, the length (L), width (W) and height (H) of each mouse were measured using vernier calipers (Mitutoyo, Japan) twice a week 10 times from the time at which the average tumor size by group reached 44.1 mm$^3$ after cancer cell transplantation to the 21st day. In addition, on the 21st day of drug administration, all subjects were killed using $CO_2$ gas, tumors were removed and appearances thereof were compared to observe differences in size and weight.

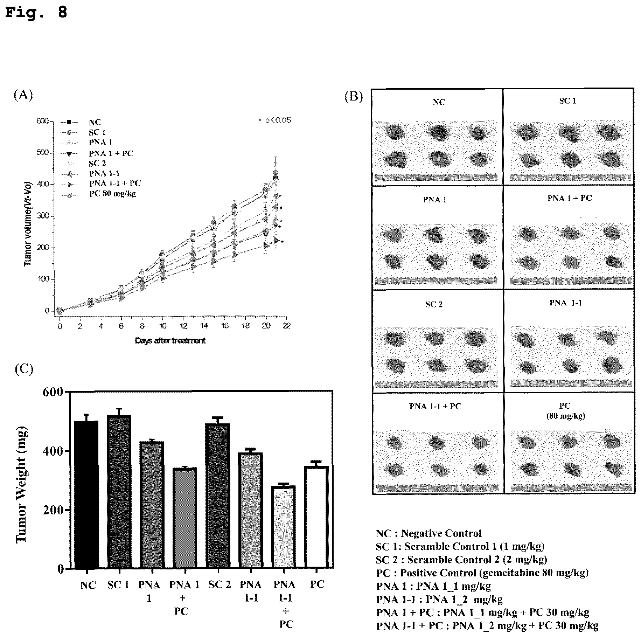

As a result, as shown in FIG. 8, statistically significant tumor size and weight reduction was observed in the group administered the nucleic acid complex (combination 1) alone or the group administered a combination of the nucleic acid complex and the positive control, compared to the control.

Example 4-2: Analysis of gene expression using Western blot assay

RIPA buffer was added at 200 $\mu$L/well to a part of the biopsied mouse tumor tissue after the experiment under the conditions of Example 4 to obtain a protein lysate. The protein was assayed from the protein lysate using a BCA assay kit (Thermo Fisher, USA), 30 $\mu$g of protein was separated by size through electrophoresis, and the protein was transferred to a PVDF membrane, treated at a ratio of 1:1000 with primary antibodies, namely, KRAS (Abcam, UK), p-ERK (Cell Signaling Technology, USA), HRAS (Abcam, UK), and NRAS (Abcam, UK), and allowed to stand at 4°C for one day.

The result was washed with 1X TBS-T, treated with secondary antibodies, namely, Goat Anti-Rabbit (Cell Signaling Technology, USA) and Mouse Anti-Goat (Santa Cruz Biotechnology, USA) at a ratio of 1:2000 and allowed to stand at room temperature for 1 hour. The result was treated with Supersignal™ West Femto Maximum Sensitivity Substrate (Thermo Fisher, USA) and efficiency of suppression of target gene expression was analyzed in pancreatic cancer tissue using an Imager 600 (Amersham, Germany).

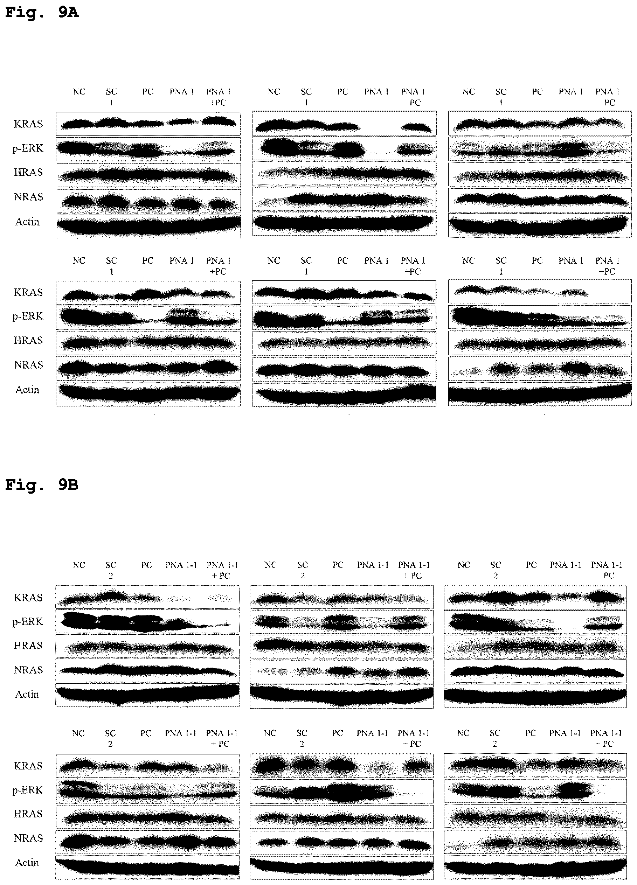

As a result, as can be seen from FIG. 9, the expression of the KRAS-targeting gene in the RAS family and the expression of the downstream gene p-ERK were decreased in the group treated with the nucleic acid complex (combination 1) compared to the control. In particular, expression of target genes and downstream genes was further inhibited in the group administered 2 mg/kg of the nucleic acid complex (combination 1) alone or the group administered a combination of the nucleic acid complex and the positive control, compared to the control (FIG. 9B).

Example 4-3: Phenotype analysis in pancreatic cancer xenograft animal models by H&E staining

Experiments were conducted under the conditions of Example 4 and tumor tissues were removed from the inoculation site of the cell line on the final day of the experiment. Tissues were fixed in a 4% formalin solution for 24 hours and then paraffin-embedded, and the tissues were sectioned into 5 um sections and mounted on slide glass. The mounted tissues were stained with Hematoxylin:Eosin staining solution for a predetermined period of time and then washed with 1X PBS, and morphological findings were analyzed under a microscope.

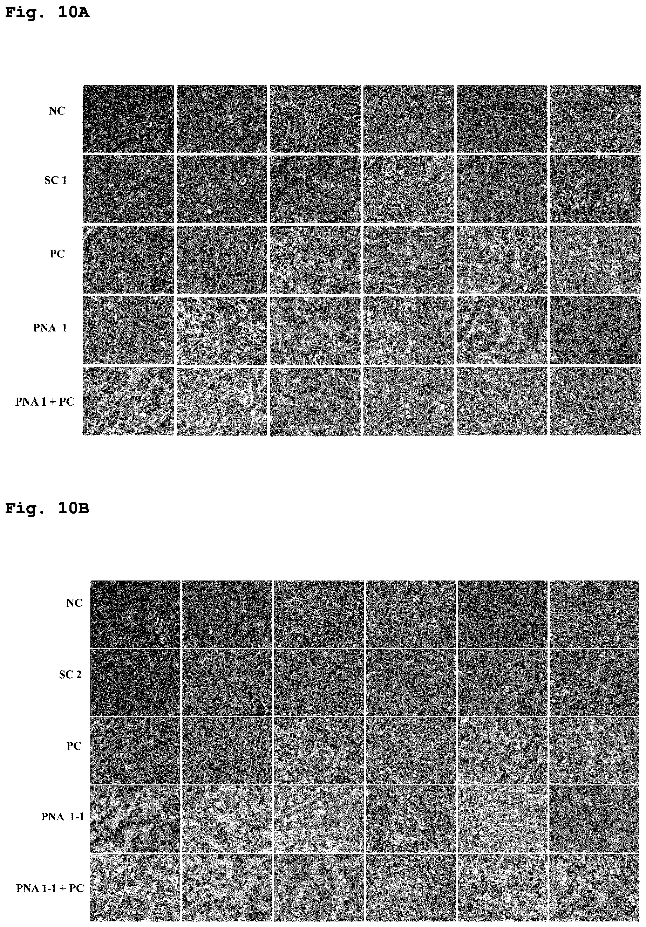

As a result, as shown in FIG. 10, it was found that the phenotype of the formed tumor was reduced in the distribution of inflammatory cells in the group treated with the nucleic acid complex (combination 1) compared to the control. In particular, it was found that the distribution and density of tumor cells were reduced in all six animals of the group administered 2 mg/kg of the nucleic acid complex (combination 1) alone, compared to the cytotoxic anticancer drug positive control (FIG. 10B).

Example 4-4: Analysis of KRAS expression changes in tissues in pancreatic cancer xenograft animal models by immunostaining

The experiment was conducted under the conditions of Example 4. On the final day of the experiment, the tumor tissue was removed from the inoculation site of the cell line and fixed in 4% formalin solution for one day, and the fixed tissue was paraffin-embedded, sectioned into 5 um sections, and mounted on a slide glass. The mounted tissue was blocked in 0.5% BSA solution for 1 hour, and allowed to react with KRAS primary antibody (Abcam, UK) at 4°C for one day. Then, the primary antibody solution was removed, washed with 1X TBS, treated with secondary antibody solution, namely Goat Anti-Rabbit (Cell Signaling Technology, USA) at a ratio of 1:2000 at room temperature for 2 hours, and then analyzed by DAB staining.

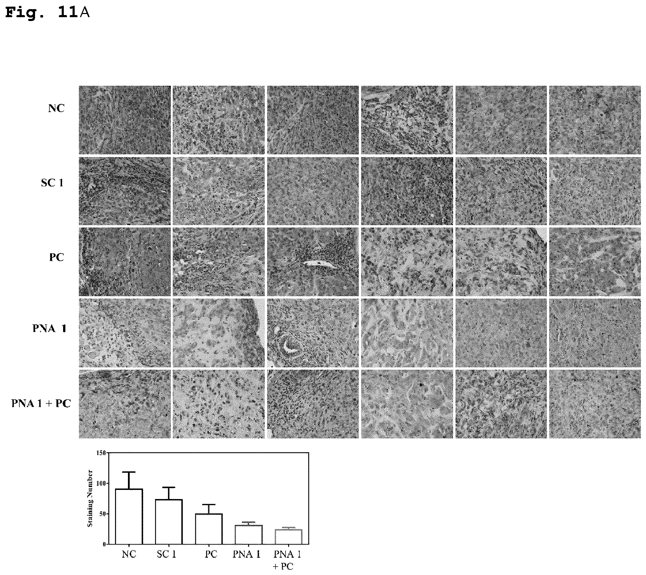

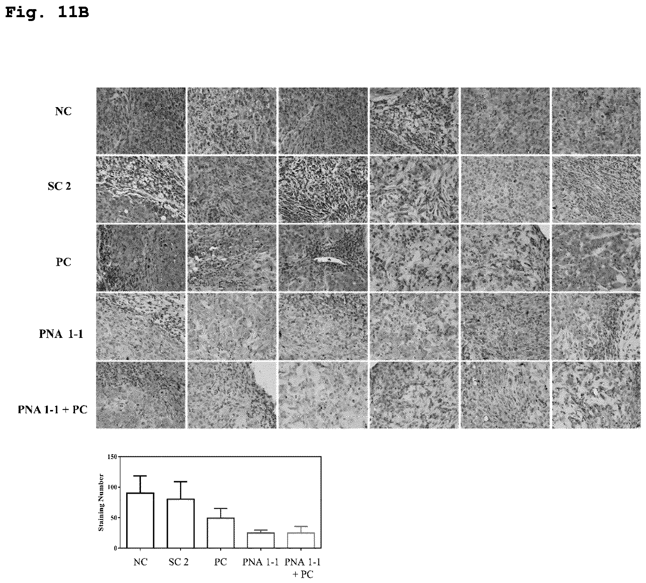

As a result, as can be seen from FIG. 11, expression of brown-stained KRAS was found in negative control (NC) and control tissues, and a significant decrease in KRAS brown reaction was observed in the group administered nucleic acid complex combination 1 alone or the group administered a combination of nucleic acid complex combination 1 and the positive control. The results were digitized and then compared. The result showed that the group treated with 2 mg/kg of nucleic acid complex combination 1 reduced KRAS expression most significantly (FIG. 11B).

Example 4-5: Analysis of apoptosis in pancreatic cancer xenograft animal models by TUNEL assay

The experiment was conducted under the conditions of Example 4. On the final day of the experiment, the tumor tissue was removed from the inoculation site of the cell line and fixed in 4% formalin solution for one day, and the fixed tissue was paraffin-embedded, sectioned into 5 um sections, and mounted on a slide glass. A reaction TdT buffer was dropped on the mounted tissue, followed by reaction at 37°C for 70 minutes. Then, the result was reacted with BrdU-Biotin antibody solution in the absence of light for 70 minutes and apoptotic cells were stained with DAB solution. After the reaction was completed, the tissue was immersed in a hematoxylin solution to stain the nuclei, washed with running water for 10 minutes, and mounted, and how much apoptosis occurred was observed under an optical microscope.

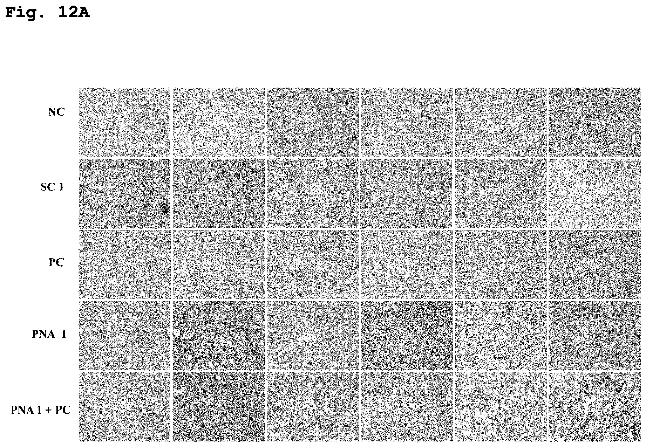

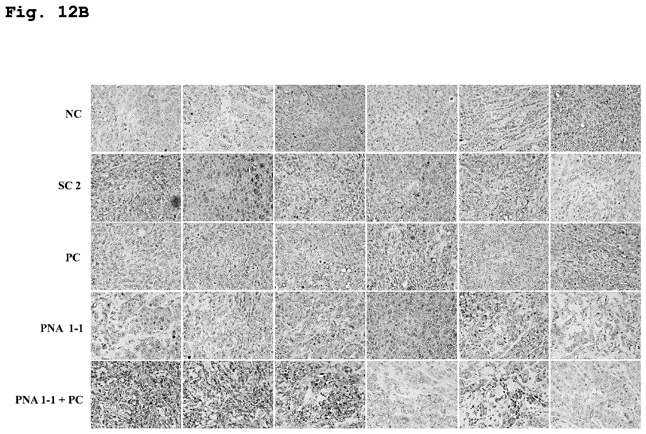

As a result, as can be seen from FIG. 12, brown-stained apoptotic cells were present in the tumor tissue and the distribution of brown-stained apoptotic cells increased in the nucleic acid complex combination 1, compared to the negative control and control. In particular, the group administered a combination of 2 mg/kg of the nucleic acid complex combination 1 and the positive control exhibited the greatest increase in the apoptotic cell response in the tissue (FIG. 12B).

**Analysis of therapeutic effects of antisense peptide nucleic acids targeting KRAS mutant G12D and G12V in KRAS mutant pancreatic cancer**

**Example 5: Bioactive peptide nucleic acid, carrier peptide nucleic acid, and preparation of complexes using the same**

G12D (in which glycine is mutated to aspartic acid) and G12V (in which glycine is mutated to valine) KRAS mutations, among genetic mutations of KRAS codon 12, which is found in about 90% of pancreatic cancer patient tissues as a target gene, were targeted to test the anticancer effect of the nucleic acid complex of the present invention on pancreatic cancer, and antisense peptide nucleic acid (antisense PNA) was used as a bioactive peptide nucleic acid for KRAS G12D and G12V mutations to determine the anticancer effect in pancreatic cancer.

The bioactive nucleic acid (antisense PNA) used as a control of the present invention has the sequence represented by SEQ ID NO: 10 and the bioactive peptide nucleic acid (antisense PNA) used to determine the pancreatic cancer anticancer effect has sequences represented by SEQ ID NOS: 8 and 9.

The nucleotide sequence, monomer modification and structure of the peptide nucleic acid-based bioactive nucleic acid used in this Example are shown in Table 4 below. All carrier peptide nucleic acids according to Examples of the present invention have sequences represented by SEQ ID NOS: 11 to 19 (Table 4).

All peptide nucleic acids used in the present invention were synthesized in PANAGENE (Korea) by HPLC purification.

[Table 4]

| Sequences of bioactive nucleic acids and carrier peptide nucleic acids to verify anti-cancer effect against pancreatic cancer | | | |
|---|---|---|---|
| Item | SEQ ID NO: | Base sequence | Monomer modification |
| Bioactive nucleic acid | 8 | 5'-AT(-)CAG(+)CT(-)CCA(+)ACT(-)AC-O-K-3' | -+-+- |
| | 9 | 5'-C(-)CTA(+)CGC(-)CAA(+)CAG(-)CT-O-K-3' | -+-+- |
| | 10 | 5'-ACT(-)CCG(+)CA(-)ATT(+)ACC(-)A-O-K-3' | -+-+- |
| Carrier peptide nucleic acid | 11 | 5'-TA(+)GTCGA(+)GGTTGA(+)TG-O-K-3' | +++ |
| | 12 | 5'-K-O-GT(+)AGTTGG(+)AGCTG(+)AT -3' | +++ |
| | 13 | 5'-TG(+)AT(+)G-O-K-3' | ++ |
| | 14 | 5'-K-O-GT(+)AG(+)T-3' | ++ |

(continued)

| Sequences of bioactive nucleic acids and carrier peptide nucleic acids to verify anti-cancer effect against pancreatic cancer | | | |
|---|---|---|---|
| Item | SEQ ID NO: | Base sequence | Monomer modification |
| | 15 | 5'-G$^{(+)}$GATGCG$^{(+)}$GTTGTC$^{(+)}$GA-O-K-3' | +++ |
| | 16 | 5'-K-O-AG$^{(+)}$CTGTTG$^{(+)}$GCGTAG$^{(+)}$G-3' | +++ |
| | 17 | 5'-GT$^{(+)}$CG$^{(+)}$A-O-K-3' | ++ |
| | 18 | 5'-K-O-AG$^{(+)}$CT$^{(+)}$G-3' | ++ |
| | 19 | 5'-TGA$^{(+)}$GGCGT$^{(+)}$TAATGG$^{(+)}$T-O-K-3' | +++ |

Table 4 above shows the sequence information of the bioactive peptide nucleic acid and carrier peptide nucleic acid used to determine the anticancer effect for pancreatic cancer targeting KRAS mutant G12D and G12V, and the sequence information of bioactive peptide nucleic acid and carrier peptide nucleic acid used as a control.

In order to impart electrical properties, modification of the monomer was performed to produce a modified peptide nucleic acid backbone designed such that the peptide nucleic acid backbone comprises lysine (Lys, K$^{(+)}$) for a positive electrical charge and the modified peptide nucleic acid backbone comprises glutamic acid (Glu, E$^{(-)}$) for a negative electrical charge.

The respective combinations of bioactive nucleic acids and carrier peptide nucleic acids were hybridized in the presence of DMSO to produce a complex comprising the bioactive nucleic acid and the carrier peptide nucleic acid.

**Example 6: Analysis of therapeutic effect for pancreatic cancer using nucleic acid complexes**

The anticancer effect for pancreatic cancer was analyzed using the nucleic acid complex comprising the carrier peptide nucleic acid and the bioactive peptide nucleic acid targeting KRAS mutant G12D and G12V prepared to have the structure of the following Table 5 according to Example 5.

Example 6-1: Cell culture

Human pancreatic cancer cell PANC-1 (KRAS G12D mutant cells) obtained from ATCC (American Type Culture Collection, USA) and CFPAC-1 (KRAS G12V mutant cells, ATCC No. 1918) were incubated at 37°C in the presence of 5% (v/v) $CO_2$ in DMEM culture medium (Dulbecco's Modified Eagle Medium, Wellgene, Korea) and IMDM culture medium (Iscove's Modified Dulbecco's Medium, Wellgene, Korea) supplemented with 10% (v/v) fetal bovine serum, 100 units/ml of penicillin, and 100 ug/ml of streptomycin, respectively.

Example 6-2: Analysis of cell viability of pancreatic cancer cell lines by MTT (MTT assay)

Pancreatic cancer cell lines PANC-1 (G12D mutation cell line) and CFPAC-1 (G12V mutation cell line) were seeded at $4 \times 10^3$ and $6 \times 10^3$, respectively, in a 96-well plate under the experimental conditions in Example 6-1, cultured for 24 hours, and treated with complexes comprising bioactive nucleic acids and carrier peptide nucleic acids prepared with the structures shown in Table 5, and the resulting cell lines were treated at a concentration of 20 pL/well with 5 mg/mL of a solution of 5 mg MTT (3-(4,5-dimethylthiazol-2-yl)-2,5-diphenyltetrazolium bromide) in 1X PBS and incubated for 4 hours, and OD (optical density) thereof was measured and analyzed with a spectrophotometer.

Nucleic acid complex combinations used in this Example are shown in Table 5 below.

[Table 5]

| Combination of nucleic acid complexes for cell viability analysis in human pancreatic cancer cell lines | |
|---|---|
| Combination | Nucleic acid complex |
| 1 | SEQ ID NOS: 8 and 11 |
| 2 | SEQ ID NOS: 8 and 12 |
| 3 | SEQ ID NOS: 8 and 13 |
| 4 | SEQ ID NOS: 8 and 14 |

(continued)

| Combination of nucleic acid complexes for cell viability analysis in human pancreatic cancer cell lines | |
| --- | --- |
| Combination | Nucleic acid complex |
| 5 | SEQ ID NOS: 9 and 15 |
| 6 | SEQ ID NOS: 9 and 16 |
| 7 | SEQ ID NOS: 9 and 17 |
| 8 | SEQ ID NOS: 9 and 18 |
| 9 | SEQ ID NOS: 10 and 19 (control) |

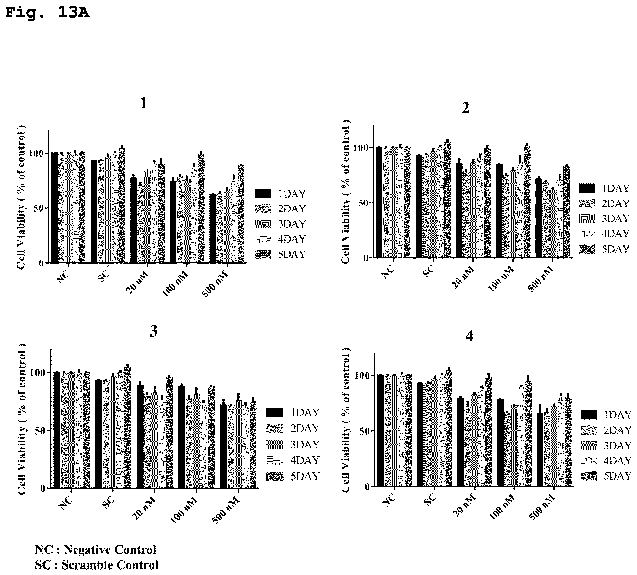

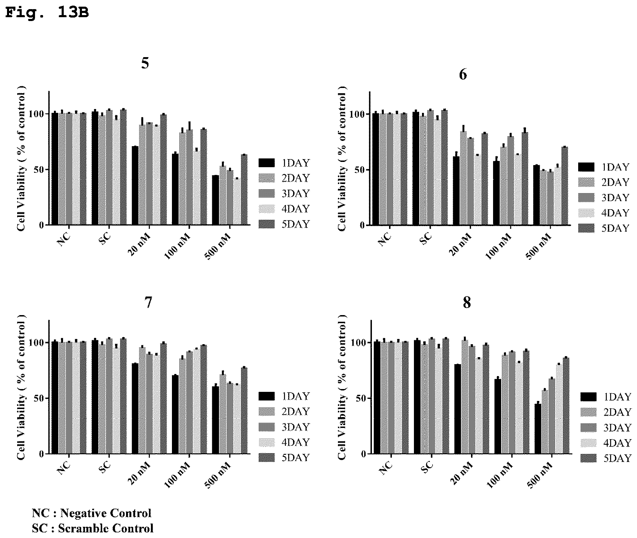

As a result, as can be seen from FIG. 13, in the pancreatic cancer cell model (FIG. 13A), nucleic acid complex combinations Nos. 1 and 2 of Table 5 decreased the cell viability in a concentration-dependent manner for up to 72 hours (FIG. 13A) and nucleic acid complex combinations Nos. 5 and 6 maintained a decrease in cell viability for up to 96 hours (FIG. 13B).

Example 6-3: Analysis of gene expression by Western blot assay

The human pancreatic cancer cell lines PANC-1 and CFPAC-1 were seeded at $1\times10^5$ in a 6-well plate and cultured for 24 hours, an experiment was performed under the conditions of Example 6-1, the cell lines were treated with a complex comprising bioactive peptide nucleic acid and carrier peptide nucleic acid, incubated for 24, 48, 72, 96, and 120 hours, and 30 $\mu$L of RIPA buffer was added to each well to obtain a protein lysate. The protein was assayed from the protein lysate using a BCA assay kit (Thermo Fisher, USA), 30 $\mu$g of protein was separated by size through electrophoresis, and the protein was transferred to a PVDF membrane, followed by treatment with primary antibodies, namely, RAS-G12D (Cell Signaling Technology, USA), RAS-G12V (Cell Signaling Technology, USA), KRAS (Abcam, UK), p-ERK (Cell Signaling Technology, USA), HRAS (Santacruz Biotechnology, USA), and NRAS (Abcam, UK) at a ratio of 1:1000 and allowing to stand at 4°C for one day.

The product was washed with 1XT BS-T, treated with, as secondary antibodies, Goat Anti-Rabbit (Cell Signaling Technology, USA), Mouse Anti-Goat (Santa Cruz Biotechnology, USA), and Goat Anti-Rat (Thermo Fisher Scientific, USA) at a ratio of 1:2,000, and allowed to stand at room temperature for 1 hour. The result was treated with Supersignal™ West Femto Maximum Sensitivity Substrate (Thermo Fisher, USA), and the efficiency of suppression of target gene expression was analyzed in pancreatic cancer cell lines using an Imager 600 (Amersham, Germany).

In this example, expression changes of RAS family (KRAS, HRAS, NRAS), G12D and G12V mutant RAS and the downstream gene p-ERK in human pancreatic cancer cell models were analyzed and the nucleic acid complex combinations used herein are shown in Table 5.

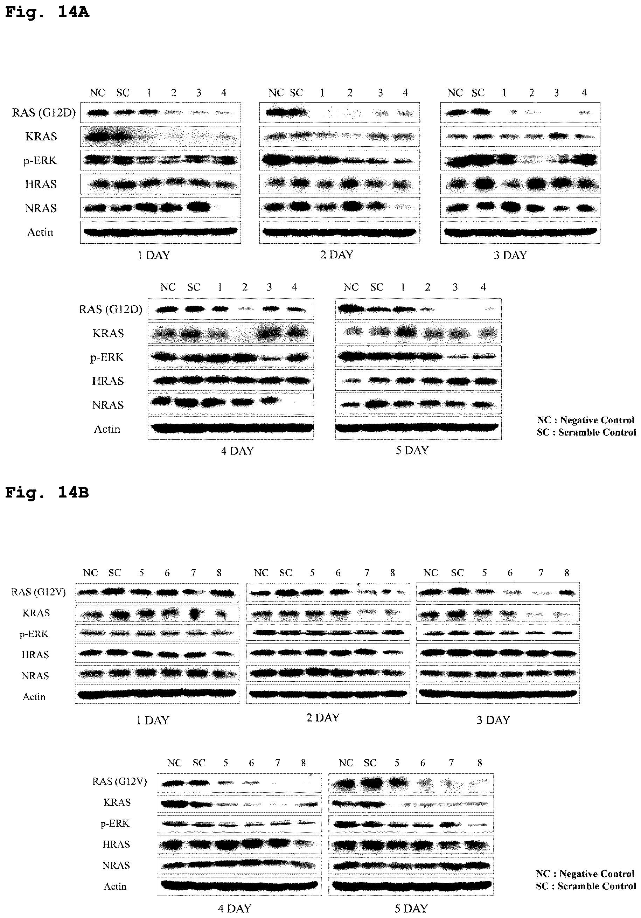

As a result, as can be seen from FIG. 14, the nucleic acid complex of combination 2 of SEQ ID NOS: 8 and 12 inhibited expression of the KRAS in the RAS family and expression of the RAS-G12D mutant gene for up to 120 hours in Bxpc-3 human pancreatic cancer cells, and inhibited expression of the downstream gene, p-EPK KRAS for up to 72 hours. In addition, the nucleic acid complex combination 5 of SEQ ID NOS: 9 and 15 and the nucleic acid complex combination 6 of SEQ ID NOS: 9 and 16 inhibited the expression of KRAS and RAS-G12V mutant genes from 48 hours to 120 hours, and also inhibited the expression of downstream gene, p-ERK from 72 hours to 120 hours. The combinations 1, 3, 4, and 8 of nucleic acid complexes exhibited effects of off-targeting unwanted sequences that reduce HRAS or NRAS in the RAS family, but combinations 2, 5, and 6 did not exhibited such effects.

**Industrial Applicability**

The nucleic acid complex according to the present invention has excellent cell penetration and intracellular activity and efficiently inhibits expression of KRAS gene and downstream genes thereof, thus being useful for prevention or treatment of pancreatic cancer.

Although specific configurations of the present invention have been described in detail, those skilled in the art will appreciate that this description is provided to set forth preferred embodiments for illustrative purposes, and should not be construed as limiting the scope of the present invention. Therefore, the substantial scope of the present invention is defined by the accompanying claims and equivalents thereto.

**Sequence List Free Text**

An electronic file is attached.

**Claims**

1.  A pharmaceutical composition for preventing, ameliorating or treating pancreatic cancer comprising, as an active ingredient, a cell-permeable nucleic acid complex in which a carrier peptide nucleic acid complementarily binds to a bioactive nucleic acid targeting a KRAS gene.

2.  The pharmaceutical composition according to claim 1, wherein the bioactive peptide nucleic acid comprises a sequence represented by SEQ ID NO: 1, 8 or 9.

3.  The pharmaceutical composition according to claim 2, wherein the bioactive peptide nucleic acid comprising the sequence represented by SEQ ID NO: 8 or 9 targets a G12D or G12V mutation of the KRAS gene.

4.  The pharmaceutical composition according to claim 1, wherein the carrier peptide nucleic acid comprises a sequence selected from the group consisting of sequences represented by SEQ ID NOS: 3 to 6 and 11 to 18.

5.  The pharmaceutical composition according to claim 1, wherein the nucleic acid complex is selected from the group consisting of:

    (i) a nucleic acid complex comprising a bioactive nucleic acid having a sequence represented by SEQ ID NO: 1 and a carrier peptide nucleic acid having a sequence represented by SEQ ID NO: 3;
    (ii) a nucleic acid complex comprising the bioactive nucleic acid having a sequence represented by SEQ ID NO: 1 and a carrier peptide nucleic acid having a sequence represented by SEQ ID NO: 4;
    (iii) a nucleic acid complex comprising a bioactive nucleic acid having a sequence represented by SEQ ID NO: 1 and a carrier peptide nucleic acid having a sequence represented by SEQ ID NO: 5;
    (iv) a nucleic acid complex comprising a bioactive nucleic acid having a sequence represented by SEQ ID NO: 1 and a carrier peptide nucleic acid having a sequence represented by SEQ ID NO: 6;
    (v) a nucleic acid complex comprising a bioactive nucleic acid having a sequence represented by SEQ ID NO: 8 and a carrier peptide nucleic acid having a sequence represented by SEQ ID NO: 11;
    (vi) a nucleic acid complex comprising a bioactive nucleic acid having a sequence represented by SEQ ID NO: 8 and a carrier peptide nucleic acid having a sequence represented by SEQ ID NO: 12;
    (vii) a nucleic acid complex comprising a bioactive nucleic acid having a sequence represented by SEQ ID NO: 8 and a carrier peptide nucleic acid having a sequence represented by SEQ ID NO: 13;
    (viii) a nucleic acid complex comprising a bioactive nucleic acid having a sequence represented by SEQ ID NO: 8 and a carrier peptide nucleic acid having a sequence represented by SEQ ID NO: 14;
    (ix) a nucleic acid complex comprising a bioactive nucleic acid having a sequence represented by SEQ ID NO: 9 and a carrier peptide nucleic acid having a sequence represented by SEQ ID NO: 15;
    (x) a nucleic acid complex comprising a bioactive nucleic acid having a sequence represented by SEQ ID NO: 9 and a carrier peptide nucleic acid having a sequence represented by SEQ ID NO: 16;
    (xi) a nucleic acid complex comprising a bioactive nucleic acid having a sequence represented by SEQ ID NO: 9 and a carrier peptide nucleic acid having a sequence represented by SEQ ID NO: 17; and
    (xii) a nucleic acid complex comprising a bioactive nucleic acid having a sequence represented by SEQ ID NO: 9 and a carrier peptide nucleic acid having a sequence represented by SEQ ID NO: 18.

6.  The pharmaceutical composition according to claim 1, wherein the bioactive nucleic acid or the carrier peptide nucleic acid comprises a material for facilitating endosomal escape that is additionally bound to a 5'-end or a 3'-end of each nucleic acid.

7.  The pharmaceutical composition according to claim 6, wherein the material for facilitating endosomal escape is at least one selected from the group consisting of peptides, lipid nanoparticles, polyplex nanoparticles, polymer nanospheres, inorganic nanoparticles, cationic lipid-based nanoparticles, cationic polymers, and pH-sensitive polymers.

8.  The pharmaceutical composition according to claim 7, wherein the peptide for facilitating endosomal escape is GLFDIIKKIAESF (SEQ ID NO: 20) or histidine(10).

9. The pharmaceutical composition according to claim 1, wherein the bioactive nucleic acid has a net negative charge or neutral.

10. The pharmaceutical composition according to claim 1, wherein the carrier peptide nucleic acid has a net positive charge.

11. The pharmaceutical composition according to claim 1, wherein the nucleic acid complex has a net positive charge.

Fig. 1A

NC : Negative Control
SC : Scramble Control

Fig. 1B

NC : Negative Control
SC : Scramble Control

**Fig. 2A**

1 DAY    2 DAY    3 DAY

4 DAY    5 DAY    SC : Scramble Control

**Fig. 2B**

1 DAY    2 DAY    3 DAY

4 DAY    5 DAY

NC : Negative Control
SC : Scramble Control

**Fig.3**

(A)

(B)

(C)

NC : Negative Control    PC : Positive Control
SC : Scramble Control    (Gemcitabine, 80 mg/kg)
                         PNA 1 : PNA 1_2mg/kg

Fig. 4

Fig. 5

Fig. 6

Fig. 7

Fig. 8

(A)

(B)

(C)

NC : Negative Control
SC 1: Scramble Control 1 (1 mg/kg)
SC 2 : Scramble Control 2 (2 mg/kg)
PC : Positive Control (gemcitabine 80 mg/kg)
PNA 1 : PNA 1_1 mg/kg
PNA 1-1 : PNA 1_2 mg/kg
PNA 1 + PC : PNA 1_1 mg/kg + PC 30 mg/kg
PNA 1-1 + PC : PNA 1_2 mg/kg + PC 30 mg/kg

Fig. 9A

Fig. 9B

Fig. 10A

Fig. 10B

Fig. 11A

Fig. 11B

Fig. 12A

Fig. 12B

NC

SC 2

PC

PNA 1-1

PNA 1-1 + PC

EP 4 265 276 A1

**Fig. 13A**

NC : Negative Control
SC : Scramble Control

Fig. 13B

NC : Negative Control
SC : Scramble Control

## Fig. 14A

## Fig. 14B

## INTERNATIONAL SEARCH REPORT

| International application No. |
|---|
| **PCT/KR2021/018964** |

| A. | CLASSIFICATION OF SUBJECT MATTER |
|---|---|

**A61K 48/00**(2006.01)i; **A61K 47/54**(2017.01)i; **A61K 47/64**(2017.01)i; **A61K 31/7088**(2006.01)i; **A61P 35/00**(2006.01)i; **C12N 15/113**(2010.01)i

According to International Patent Classification (IPC) or to both national classification and IPC

| B. | FIELDS SEARCHED |
|---|---|

Minimum documentation searched (classification system followed by classification symbols)

A61K 48/00(2006.01); A61K 38/10(2006.01); A61K 47/54(2017.01); A61K 47/64(2017.01); C07K 7/08(2006.01); C12N 15/113(2010.01); C12Q 1/68(2006.01); C12Q 1/6883(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: KRAS, 생활성 펩티드 핵산(bioactive peptide nucleic acid), 캐리어 펩티드 핵산 (carrier peptide nucleic acid), 췌장암(pancreas cancer), 엔도좀 탈출(endosome escape), 핵산 복합체(nucleic acid complex)

| C. | DOCUMENTS CONSIDERED TO BE RELEVANT |
|---|---|

| Category* | Citation of document, with indication, where appropriate, of the relevant passages | Relevant to claim No. |
|---|---|---|
| DY | KR 10-2019-0096148 A (SEASUN THERAPEUTICS) 19 August 2019 (2019-08-19)<br>See claims 1-4, 9, 25 and 26. | 1,6-11 |
| DA | | 2-5 |
| Y | KR 10-2015-0006477 A (GRADALIS, INC.) 16 January 2015 (2015-01-16)<br>See abstract; claims 1 and 34; and paragraphs [0018], [0140] and [0141]. | 1,6-11 |
| Y | KR 10-2020-0090419 A (SEASUN THERAPEUTICS) 29 July 2020 (2020-07-29)<br>See claims 1 and 4-8. | 1,6-11 |
| Y | KR 10-2018-0018405 A (SEASUN BIOMATERIALS) 21 February 2018 (2018-02-21)<br>See claims 1 and 22-24. | 1,9-11 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **23 March 2022** | **23 March 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
| --- |
| **PCT/KR2021/018964** |

## C. DOCUMENTS CONSIDERED TO BE RELEVANT

| Category* | Citation of document, with indication, where appropriate, of the relevant passages | Relevant to claim No. |
| --- | --- | --- |
| A | US 2018-0312542 A1 (PRESIDENT AND FELLOWS OF HARVARD COLLEGE) 01 November 2018 (2018-11-01) See entire document. | 1-11 |

Form PCT/ISA/210 (second sheet) (July 2019)

# EP 4 265 276 A1

**INTERNATIONAL SEARCH REPORT**

| International application No. |
|---|
| **PCT/KR2021/018964** |

**Box No. I    Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

   a.  ☑  forming part of the international application as filed:

   ☑  in the form of an Annex C/ST.25 text file.

   ☐  on paper or in the form of an image file.

   b.  ☐  furnished together with the international application under PCT Rule 13*ter*.1(a) for the purposes of international search only in the form of an Annex C/ST.25 text file.

   c.  ☐  furnished subsequent to the international filing date for the purposes of international search only:

   ☐  in the form of an Annex C/ST.25 text file (Rule 13*ter*.1(a)).

   ☐  on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (July 2019)

INTERNATIONAL SEARCH REPORT
Information on patent family members

International application No.

**PCT/KR2021/018964**

| Patent document cited in search report | | | Publication date (day/month/year) | Patent family member(s) | | | Publication date (day/month/year) |
|---|---|---|---|---|---|---|---|
| KR | 10-2019-0096148 | A | 19 August 2019 | AU | 2019-219472 | A1 | 10 September 2020 |
| | | | | CA | 3090754 | A1 | 15 August 2019 |
| | | | | CN | 112135907 | A | 25 December 2020 |
| | | | | EP | 3750995 | A1 | 16 December 2020 |
| | | | | JP | 2021-515589 | A | 24 June 2021 |
| | | | | KR | 10-2262260 | B1 | 09 June 2021 |
| | | | | US | 2021-0171953 | A1 | 10 June 2021 |
| | | | | WO | 2019-156365 | A1 | 15 August 2019 |
| KR | 10-2015-0006477 | A | 16 January 2015 | AR | 091004 | A1 | 30 December 2014 |
| | | | | AU | 2013-259387 | A1 | 20 November 2014 |
| | | | | AU | 2013-259387 | B2 | 15 December 2016 |
| | | | | CA | 2872930 | A1 | 14 November 2013 |
| | | | | CN | 104685055 | A | 03 June 2015 |
| | | | | EP | 2847333 | A1 | 18 March 2015 |
| | | | | IL | 235486 | A | 31 December 2014 |
| | | | | JP | 2015-521040 | A | 27 July 2015 |
| | | | | JP | 2017-221198 | A | 21 December 2017 |
| | | | | JP | 6283661 | B2 | 21 February 2018 |
| | | | | MX | 2014013475 | A | 15 May 2015 |
| | | | | SG | 11201407239 | A | 30 December 2014 |
| | | | | TW | 201402813 | A | 16 January 2014 |
| | | | | US | 2013-0302407 | A1 | 14 November 2013 |
| | | | | US | 2016-0333351 | A1 | 17 November 2016 |
| | | | | US | 9353373 | B2 | 31 May 2016 |
| | | | | WO | 2013-170071 | A1 | 14 November 2013 |
| KR | 10-2020-0090419 | A | 29 July 2020 | KR | 10-2156324 | B1 | 15 September 2020 |
| KR | 10-2018-0018405 | A | 21 February 2018 | AU | 2017-310146 | A1 | 28 March 2019 |
| | | | | AU | 2017-310146 | B2 | 17 December 2020 |
| | | | | CA | 3033474 | A1 | 15 February 2018 |
| | | | | CN | 109804070 | A | 24 May 2019 |
| | | | | EP | 3498844 | A1 | 19 June 2019 |
| | | | | JP | 2019-526624 | A | 19 September 2019 |
| | | | | JP | 6818889 | B2 | 20 January 2021 |
| | | | | KR | 10-1963885 | B1 | 01 April 2019 |
| | | | | KR | 10-2018-0100523 | A | 11 September 2018 |
| | | | | US | 10745446 | B2 | 18 August 2020 |
| | | | | US | 2019-0185519 | A1 | 20 June 2019 |
| | | | | WO | 2018-030789 | A1 | 15 February 2018 |
| US | 2018-0312542 | A1 | 01 November 2018 | WO | 2017-070182 | A1 | 27 April 2017 |

Form PCT/ISA/210 (patent family annex) (July 2019)

**EP 4 265 276 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 2017008636 W

- KR 1020190096148

**Non-patent literature cited in the description**

- **ANDREW M. WATERS et al.** *Cold Spring Harb Perspect Med.,* 2018, vol. 8 (9), a031435
- **KIRSTEN L.** *Trends Biochem Sci.,* 2014, vol. 39 (2), 91-100
- **LOUIS BUSCAIL.** *Nature Rev Gastroenterol Hepatol,* 2020, vol. 17, 153-168

- **D. W. PACK ; A. S. HOFFMAN ; S. PUN ; P. S. STAYTON.** Design and development of polymers for gene delivery. *Nat. Rev. Drug. Discov.,* 2005, vol. 4, 581-593